# EUROPÄISCHE PATENTSCHRIFT

(11) **EP 2 515 963 B1**
(45) Veröffentlichungstag und Bekanntmachung des Hinweises auf die Patenterteilung: **14.02.2024**
(21) Anmeldenummer: 10795244.2
(22) Anmeldetag: 14.12.2010
(51) Int. Cl.: A61M 1/16, A61M 1/28, A61M 1/14

(54) **DIALYSEMASCHINE, INSBESONDERE PERITONEALDIALYSEMASCHINE**
DIALYSIS MACHINE, IN PARTICULAR PERITONEAL DIALYSIS MACHINE
MACHINE DE DIALYSE, EN PARTICULIER MACHINE DE DIALYSE PÉRITONÉALE

(30) Priorität: 23.12.2009 DE 102009060330
(43) Veröffentlichungstag der Anmeldung: 31.10.2012
(73) Patentinhaber: Fresenius Medical Care Deutschland GmbH, 61352 Bad Homburg (DE)
(72) Erfinder: HEDMANN, Frank, 97332 Volkach (DE); SEBESTA, Sven, 97421 Schweinfurt (DE)
(74) Vertreter: Laufhütte, Dieter
(86) Internationale Anmeldenummer: PCT/EP2010/007631
(87) Internationale Veröffentlichungsnummer: WO 2011/079907

(56) Entgegenhaltungen:
- EP-A1- 1 669 051
- WO-A1-93/09820
- WO-A1-2008/106452
- WO-A1-2009/108405
- WO-A2-2007/144427
- WO-A2-2009/006471
- GB-A- 2 099 391
- GB-A- 2 362 843
- US-A1- 2009 009 179
- US-A1- 2009 012 452

## Beschreibung

Die vorliegende Erfindung betrifft eine Dialysemaschine, insbesondere eine Peritonealdialysemaschine, an welcher ein Fluidsystem mit einem Mehrkammerbehälter mit mindestens zwei durch eine mechanisch zu öffnende Trennanordnung getrennten Kammern mit Einzellösungen ankoppelbar ist. Die Dialysemaschine weist dabei weiterhin eine Steuerung und mindestens einen Sensor zur Bestimmung einer Messgröße im Fluidsystem auf.

Im Bereich der Dialyse, insbesondere im Bereich der Peritonealdialyse, ist es üblich, vorbereitete Einzellösungen erst kurz vor der Behandlung miteinander zu vermischen, um so eine fertige Dialyselösung zur Verfügung zu stellen. Dies liegt zum einen daran, dass bestimmte Inhaltstoffe lagerunverträglich sind und bei Lagerung in gemischtem Zustand ungewollte Abfallprodukte entstehen. Zudem ist die Hitzesterilisation der Lösung in gemischtem Zustand oftmals problematisch oder nicht möglich.

Um eine einfache Handhabung solcher lagerunverträglichen Lösungen zu gewährleisten, werden diese in lagerverträgliche Einzellösungen unterteilt und in einem Mehrkammerbehälter, welcher mindestens zwei Kammern aufweist, verpackt. Um eine einfache Vereinigung der Einzellösungen zu gewährleisten, sind die Kammern durch eine Trennanordnung getrennt, welche mechanisch geöffnet werden kann. Kurz vor der Behandlung wird die Trennanordnung also manuell vom Patienten oder einer Bedienperson geöffnet, so dass sich die Einzellösungen zu der gewünschten Dialyselösung vermischen.

Als Merhkammerbehälter kommt dabei üblicherweise ein Mehrkammerbeutel zum Einsatz. Als Trennanordnung zur Aufteilung des Beutels in mehrere getrennte Kammern wird üblicherweise eine leicht zu öffnende Naht, welche auch Peel-Naht genannt wird, eingesetzt. Die Naht ist dabei so stabil ausgeführt, dass eine unbeabsichtigte Öffnung der Naht und eine damit verbundene Vereinigung der Einzellösung während der Lagerung und des Transportes ausgeschlossen werden kann. Kurz vor der Behandlung wird dann die Peel-Naht zwischen den Kammern geöffnet, so dass sich die mindestens zwei Kammern zu einer einzigen Kammer vereinigen und die vorher getrennt gelagerten Einzellösungen sich miteinander vermischen können. Die Öffnung erfolgt dabei durch den Patienten bzw. einer Benutzer, indem Druck auf mindestens eine der Kammern ausgeübt wird, wodurch die Peel-Naht geöffnet wird.

Nach dem Öffnen der Trennanordnung kann nun Dialyseflüssigkeit aus dem Mehrkammerbehälter entnommen werden. Hierzu weist der Mehrkammerbehälter üblicherweise einen Ablauf z.B. in Form einer Schlauchanordnung auf. Dieser ist bei bekannten Mehrkammerbehältern, insbesondere bei Mehrkammerbeuteln, üblicherweise im Bereich einer der mindestens zwei Kammern angeordnet. Wird daher die Trennanordnung nicht sachgerecht geöffnet, so fließt lediglich eine der Einzellösungen aus dem Mehrkammerbehälter ab. Dies kann zu erheblichen Risiken für den Patienten führen, da hierdurch nicht die korrekte Dialyseflüssigkeit, welche aus einer Mischung der mehreren Einzellösungen besteht, sondern eine Einzellösung eingesetzt wird.

Die GB 2 362 843 A offenbart eine Hämodialysemaschine, an welche ein Fluidsystem mit einem Mehrkammerbehälter mit mindestens zwei durch eine mechanisch zu öffnende Trennanordnung getrennten Kammern mit Einzellösungen angekoppelt ist. Die Trennanordnung zwischen den Kammern des Mehrkammerbeutels soll durch den Anwender geöffnet werden.

Die WO 93/09820 A1 offenbart einen Mehrkammerbeutel für die Peritonealdialyse, wobei eine Teillösung umfassend Glukosemoleküle von einer weiteren Teillösung getrennt angeordnet ist. Die Teillösungen sind durch eine mechanisch zu öffnende Naht voneinander getrennt.

Aus dem Bereich der Hämodialyse ist es daher bekannt, auf den Einsatz von Mehrkammerbehältern zu verzichten und die Gesamtlösung durch getrenntes Abpumpern der Einzellösungen und ein automatisches Mischen der Einzellösungen durch die Dialysemaschine herzustellen. Hier wird üblicherweise ein Leitfähigkeitssensor eingesetzt, welcher die korrekte Mischung der entstehenden Gesamtlösung überwacht.

Im Bereich der Peritonealdialyse wird das Dialysat jedoch direkt dem Bauchraum des Patienten zugeführt, so dass erheblich höhere Anforderungen an die Sterilität des Dialysats zu stellen sind. Der Einsatz von Leitfähigkeitssensoren ist im Bereich der Peritonealdialyse daher äußerst problematisch. Daher werden gerade im Bereich der Peritoenaldialyse Mehrkammerbehälter mit einer mechanisch zu öffnenden Trennanordnung eingesetzt.

Bei bekannten Dialysesystemen, bei welchen Mehrkammerbehälter zum Einsatz kommen, wird daher z.B. über eine Bedienerführung mit Warnhinweisen versucht, den Bediener zur sachgerechten Öffnung der Trennanordnung anzuleiten. Zudem sind teilweise mechanische Konstruktionen des Mehrkammerbehälters bekannt, bei welchen ein Ablassen von Flüssigkeit aus dem Mehrkammerbehälter erst nach Öffnung der Trennanordnung möglich wird.

Im Stand der Technik sind auch mehrere Dialysesysteme bekannt, die Mittel zur Bestimmung der sachgerechten Zusammensetzung der Dialyselösung aufweisen. So offenbart die US 2009/009179 A1 eine Dialysemaschine, wobei zur Bestimmung der sachgerechten Öffnung der Trennanordnung zwischen den Kammern des Mehrkammerbeutels eine Messung der elektrischen Kapazität erfolgt. Die WO 2008/106452 A1 offenbart eine Kontrolle mit Hilfe einer Temperaturmessung und einer Leitfähigkeitsmessung. In der WO 2009/108405 A1 werden in diesem Zusammenhang eine Kapazitätsmessung, eine Druckmessung, eine Ultraschallmessung, oder eine optische Messung vorgeschlagen. Ein in der WO 2009/006471 A2 vorgestelltes Gerät weist einen induktiven Sensor auf, um eine sachgerechte Öffnung der Trennanordnung zu detektieren. Die US 2009/012452 A1 offenbart ein Gerät, in dem eine Leitfähigkeitsmessung in der zum Patienten führenden Leitung zur Anwendung kommt, um zu ermitteln, ob eine sachgerechte Zusammensetzung der Dialyselösung vorliegt.

Eine ausreichende Sicherheit konnte mit bekannten Systemen bisher jedoch nicht erreicht werden.

Aufgabe der vorliegenden Erfindung ist es, die durch den Einsatz eines Mehrkammerbehälters mit einer mechanisch zu öffnenden Trennanordnung entstehenden Gefahren für den Patienten zu verringern.

Diese Aufgabe wird erfindungsgemäß durch eine Dialysemaschine gemäß Anspruch 1 gelöst. Die erfindungsgemäße Dialysemaschine ist an ein Fluidsystem mit einem Mehrkammerbehälter mit mindestens zwei durch eine mechanisch zu öffnende Trennanordnung getrennten Kammern mit Einzellösungen ankoppelbar. Insbesondere handelt es sich dabei um eine Trennanordnung, welche manuell durch den Patienten oder eine Bedienperson geöffnet werden kann bzw. muss. Weiterhin weist die Dialysemaschine eine Steuerung mit mindestens einem Sensor zur Bestimmung einer Messgröße im Fluidsystem auf. Erfindungsgemäß ist dabei vorgesehen, dass die Steuerung eine Vorrichtung zur automatischen Überprüfung der sachgerechten Öffnung der Trennanordnung des Mehrkammerbehälters aufweist, welche die sachgerechte Öffnung der Trennanordnung eines Mehrkammerbehälters eines an die Dialysemaschine angekoppelten Fluidsystems anhand der vom Sensor bestimmten Messgröße automatisch überprüft. Durch die automatische Überprüfung der sachgerechten Öffnung der Trennanordnung durch die Dialysemaschine können die mit der mechanisch zu öffnenden Trennanordnung einhergehenden Risiken für den Patienten minimiert werden. Hierdurch können die Vorteile eines Mehrkammerbehälters genutzt werden, ohne die bisher damit verbundenen Risiken in Kauf nehmen zu müssen.

Vorteilhafterweise überprüft die erfindungsgemäße Vorrichtung zur automatischen Überprüfung dabei die sachgerechte Öffnung der Trennanordnung des Mehrkammerbehälters, bevor Flüssigkeit aus dem Mehrkammerbehälter für die Dialysebehandlung herangezogen wurde. Für die Peritonealdialyse bedeutet dies, dass die sachgerechte Öffnung der Trennanordnung überprüft wird, bevor Flüssigkeit aus dem Mehrkammerbehälter zum Patienten geleitet wird. Hierdurch kann ausgeschlossen werden, dass bei fehlerhafter Öffnung eine nicht korrekt gemischte Flüssigkeit für die Dialysebehandlung verwendet wird.

Vorteilhafterweise startet die Steuerung dabei die Dialysebehandlung nur dann, wenn eine sachgerechte Öffnung der Trennanordnung erkannt wurde. In weiterhin vorteilhafter Weise gibt die Steuerung ein Signal an den Benutzer aus, wenn eine nicht sachgerechte Öffnung der Trennanordnung erkannt wurde. Hierdurch wird der Benutzer darauf aufmerksam gemacht, dass die Trennanordnung noch sachgerecht geöffnet werden muss.

In weiterhin vorteilhafter Weise ist vorgesehen, dass der Sensor die Messgröße im Fluidsystem ohne direkten Kontakt mit dem Fluid im Fluidsystem misst. Hierdurch kann der direkte Kontakt von Komponenten der Dialysemaschine mit dem Fluid im Fluidsystem vermieden werden. Dies ist insbesondere bei der Peritonealdialyse von großer Wichtigkeit, um die Sterilität des Fluids im Fluidsystem sicherzustellen. Weiterhin kann hierdurch das komplette Fluidsystem als ein Einmalartikel ausgeführt werden.

Vorteilhafterweise ist der Sensor dabei in der Dialysemaschine angeordnet und wird beim Ankoppeln des Fluidsystems an einen Sensorbereich des Fluidsystems angekoppelt. Der Sensor kann so eine Messgröße im Fluidsystem bestimmen, ohne in direkten Kontakt mit der Flüssigkeit im Fluidsystem zu kommen.

Vorteilhafterweise handelt es sich dabei erfindungsgemäß um einen Temperatursensor und/oder einen Drucksensor und/oder einen Gewichtsensor und/oder einen optischen Sensor. All diese Sensoren müssen nicht direkt mit dem Fluid im Fluidsystem in Kontakt kommen, um eine Messgröße des Fluidsystems zu bestimmen. Insbesondere kann dabei ein Temperaturwert, ein Druckwert, ein Gewichtswert oder eine optische Eigenschaft im Fluidsystem bestimmt werden.

Dabei kann vorgesehen sein, dass die erfindungsgemäße Dialysemaschine die sachgerechte Öffnung der Trennanordnung des Mehrkammerbeutels anhand eines Meßwertes bestimmt, insbesondere anhand einer Abfrage, ob ein Meßwert einen bestimmten Grenzwert überschreitet und/oder einen bestimmten Grenzwert unterschreitet.

Alternativ oder zusätzlich kann vorgesehen sein, dass die Vorrichtung zur automatischen Überprüfung die sachgerechte Öffnung der Trennanordnung des Mehrkammerbeutels anhand der Veränderung der vom Sensor bestimmten Messgröße über die Zeit automatisch überprüft. Hierfür wird die Messgröße zu mindestens zwei unterschiedlichen Zeitpunkten bestimmt. Vorteilhafterweise kann die Messgröße auch im wesentlichen kontinuierlich bestimmt und die Ableitung der Messgröße nach der Zeit berechnet werden.

Vorteilhafterweise erfolgt die Überprüfung der sachgerechten Öffnung dabei anhand einer Abfrage, ob die Veränderung einen gewissen Grenzwert überschreitet und/oder ob die Veränderung einen gewissen Grenzwert unterschreitet.

Vorteilhafterweise wirkt die Dialysemaschine dabei vor Bestimmung mindestens eines Meßwertes auf das Fluidsystem ein, insbesondere thermisch und/oder mechanisch. In weiterhin vorteilhafter Weise kann ein erster Meßwert vor einer solchen Einwirkung und ein zweiter Meßwert während oder nach einer solchen Einwirkung auf das Fluidsystem bestimmt werden. Die Einwirkung auf das Fluidsystem erfolgt dabei vorteilhafterweise über ein Heizelement, einen Ventilaktor und/oder einen Pumpaktor der Dialysemaschine.

In einer Ausführungsform der Erfindung ist der Sensor an einem Bereich des Fluidsystems angeordnet, durch welchen Flüssigkeit, die aus dem Mehrkammerbehälter abgeführt wurde, hindurchfließt, insbesondere an einem Schlaubereich oder an einem Kassettenbereich des Fluidsystems. Die Dialysemaschine öffnet daher zunächst eines oder mehrere Ventile im Fluidsystem und überprüft dann eine Eigenschaft der aus dem Mehrkammerbeutel in das übrige Fluidsystem einfließenden Flüssigkeit.

Alternativ kann der Sensor auch eine Eigenschaft der im Merkammerbehälter befindlichen Flüssigkeit messen.

Anhand der Messgröße kann dabei erfindungsgemäß festgestellt werden, ob die Trennanordnung sachgerecht geöffnet wurde. Insbesondere wird dabei vorteilhafterweise eine physikalische Eigenschaft des Fluidsystems bestimmt, welche durch die Öffnung der Trennanordnung beeinflußt wird.

In weiterhin vorteilhafter Weise kann vorgesehen sein, dass die Vorrichtung zur automatischen Überprüfung der sachgerechten Öffnung der Trennanordnung des Mehrkammerbeutels auch die sachgerechte Mischung der Einzellösungen überprüft. Die sachgerechte Mischung erfolgt dabei üblicherweise, indem der Behälter nach Öffnung der Trennanordnung entsprechend bewegt wird. Allerdings erfolgt die Mischung bei geöffneter Trennanordnung auch automatisch durch Diffusion, so dass die erfindungsgemäße Vorrichtung zur automatischen Überprüfung der sachgerechten Öffnung der Trennanordnung nicht zwingend die sachgerechte Mischung der Einzellösungen direkt überprüfen muss. Die Sicherheit wird jedoch offensichtlich erhöht, wenn die Messgröße nicht nur von der Öffnung der Trennanordnung, sondern auch von der Vermischung der Einzellösung abhängt.

Erfindungsgemäß weist die Dialysemaschine eine Heizung zum Erwärmen der Flüssigkeit im Mehrkammerbehälter auf, wobei zur Überprüfung der sachgerechten Öffnung der Trennanordnung des Mehrkammerbehälters eine erste Kammer des Mehrkammerbehälters beheizt wird und eine zweite Kammer nicht beheizt wird, wobei die Überprüfung durch Bestimmung einer Temperatur und/oder einer Temperaturveränderung im Fluidsystem erfolgt.

Insbesondere weist die Heizung der Dialysemaschine dabei zwei getrennte Heizbereiche auf, welchen zwei Kammern des Mehrkammerbehälters zugeordnet sind. Zur Überprüfung der sachgerechten Öffnung der Trennanordnung wird nun für einen bestimmten Zeitraum nur eine der beiden Heizbereiche und damit nur eine der beiden Kammern beheizt. Durch Bestimmung der Temperatur bzw. der Temperaturveränderung in mindestens einer der Kammern während bzw. nach diesem Heizvorgang kann nun bestimmt werden, ob die Trennanordnung ordnungsgemäß geöffnet wurde und die beiden Kammern fluidisch verbunden sind.

Die Heizung der Dialysemaschine weist dabei vorteilhafterweise eine Heizfläche auf, auf welche der Mehrkammerbehälter, insbesondere der Mehrkammerbeutel gelegt werden kann. Die Heizfläche weist dabei mindestens zwei Heizbereiche auf, welche getrennt voneinander beheizt werden können und auf welche die entsprechenden Kammern des Mehrkammerbehälters gelegt werden.

In weiterhin vorteilhafter Weise weist die Dialysemaschine einen der ersten Kammer zugeordneten Temperatursensor auf, über welchen die Temperatur der Flüssigkeit im Bereich der ersten Kammer bestimmbar ist. Alternativ oder zusätzlich weist die Dialysemaschine einen der zweiten Kammer zugeordneten Temperatursensor auf, über welchen die Temperatur der Flüssigkeit im Bereich der zweiten Kammer bestimmbar ist. Insbesondere kann der entsprechende Sensor dabei auf der Heizfläche der Heizung angeordnet und mit dem entsprechenden Bereich des Mehrkammerbehälters in Kontakt stehen. Alternativ kann auch die Temperatur der aus der ersten bzw. der zweiten Kammer des Mehrkammerbehälters abfließenden Flüssigkeit bestimmt werden.

Vorteilhafterweise erfolgt die Überprüfung der ordnungsgemäßen Öffnung der Trennanordnung dabei durch eine Abfrage, ob die Temperatur der Flüssigkeit in der zweiten Kammer eine gewisse Temperaturschwelle überschritten hat und/oder ob die Veränderung der Temperatur der Flüssigkeit in der zweiten Kammer über einem gewissen Grenzwert liegt. Ist die Trennanordnung nicht sachgerecht geöffnet, so stehen die beiden Kammern nicht ausreichend fluidisch in Verbindung. Hierdurch wird durch die Erwärmung des Bereiches der ersten Kammer die Flüssigkeit in der zweiten Kammer nicht oder nur wenig erwärmt.

Alternativ oder zusätzlich erfolgt die Überprüfung durch eine Abfrage, ob die Temperatur der Flüssigkeit in der ersten Kammer unterhalb einer gewissen Temperaturschwelle geblieben ist und/oder ob die Veränderung der Temperatur der Flüssigkeit in der ersten Kammer unter einem gewissen Grenzwert liegt. Wurde die Trennanordnung nicht sachgerecht geöffnet, so liegt keine ausreichende fluidische Verbindung zwischen den beiden Kammern vor. Hierdurch ist die Flüssigkeitsmenge, welche von der Heizung im Bereich der ersten Kammer erwärmt wird, entsprechend geringer. Hierdurch erwärmt sich die Flüssigkeit im Bereich der ersten Kammer schneller. Dies kann wiederum zum Erkennen einer nicht ordnungsgemäßen Öffnung der Trennanordnung herangezogen werden.

In einer bevorzugten Ausführung der vorliegenden Erfindung erfolgt die Überprüfung der sachgerechten Öffnung der Trennanordnung auch durch Bestimmung einer optischen Eigenschaft der Flüssigkeit im Fluidsystem. Auch hierdurch kann über einen entsprechenden optischen Sensor die ordnungsgemäße Öffnung der Trennanordnung überprüft werden.

Vorteilhafterweise sind die Einzellösungen dabei so ausgeführt, dass sich eine optische Eigenschaft einer ersten Einzellösung durch die Vermischung mit einer zweiten Einzellösung verändert. Die Bestimmung der optischen Eigenschaft kann dabei ebenfalls ohne einen direkten Kontakt des Sensors mit dem Fluid erfolgen. Weiterhin erlaubt die Bestimmung der optischen Eigenschaft der Flüssigkeit auch eine Verifikation, ob eine ordnungsgemäße Vermischung der Flüssigkeiten stattgefunden hat.

Vorteilhafterweise wird erfindungsgemäß als optische Eigenschaft die Farbe und/oder die Helligkeit und/oder die Polarisierungseigenschaften der Flüssigkeit bestimmt, welche sich im Mehrkammerbehälter befindet oder aus dem Mehrkammerbehälter abfließt.

Vorteilhafterweise kann vorgesehen sein, dass sich die Farbe und/oder Trübung einer ersten Einzellösung durch die Vermischung mit einer zweiten Einzellösung ändert. Insbesondere kann dabei vorgesehen sein, dass mindestens eine der Einzellösungen im Mehrkammerbehälter eingefärbt ist, so dass sich durch die Mischung mit einer anderen Einzellösung eine Farbveränderung mindestes einer der Einzellösungen ergibt. Vorteilhafterweise ist dabei die Einzellösung in der Kammer, in deren Bereich der Ablauf zur Entnahme der Gesamtlösung angeordnet ist, nicht eingefärbt, während eine zweite Einzellösung in einer weiteren Kammer eingefärbt ist. Wird die Trennanordnung nicht sachgerecht geöffnet, so findet keine Vermischung statt und die abfließende Lösung ist weiterhin klar. Wurde die Trennanordnung dagegen geöffnet, so ist die Gesamtlösung eingefärbt. Die sachgerechte Öffnung kann daher z.B. über einen Trübungssensor erkannt werden.

Alternativ oder zusätzlich kann vorgesehen sein, dass sich die Polarisierungseigenschaften einer ersten Einzellösung durch die Vermischung mit einer zweiten Einzellösung ändern. Der Sensor bestimmt dann vorteilhafterweise die Polarisierungseigenschaften der Flüssigkeit im Mehrkammerbeutel bzw. vorteilhafterweise der aus dem Mehrkammerbeutel abfließenden Flüssigkeit.

Einsetzbar ist hierbei ein Mehrkammerbehälter mit mindestens zwei durch eine mechanisch zu öffnende Trennanordnung getrennten Kammern. Insbesondere kann eine Trennanordnung dabei manuell durch den Patienten oder eine Bedienperson geöffnet werden. Insbesondere umfasst der Mehrkammerbeutel mindestens zwei durch eine mechanisch zu öffnende Peel-Naht getrennte Kammern. Dabei ist vorgesehen, dass die beiden Kammern unterschiedliche Einzellösungen enthalten, und sich eine optische Eigenschaft einer ersten Einzellösung durch Mischung mit einer zweiten Einzellösung ändert.

Insbesondere kann es sich bei einer optischen Eigenschaft um die Farbe der Einzellösung handeln. Insbesondere ist dabei nur eine zweite Einzellösung, welche sich in einer Kammer befindet, in welcher der Ablauf nicht angeordnet ist, eingefärbt. Bei dem Farbstoff handelt es sich dabei um einen gesundheitlich unbedenklichen Farbstoff.

Alternativ oder zusätzlich kann vorgesehen sein, dass die optische Eigenschaft, welche sich durch die Mischung mit der zweiten Einzellösung ändert, eine Polarisationseigenschaften der Einzellösung ist. Hierdurch kann durch Bestimmung dieser Polarisationseigenschaft die sachgerechte Öffnung der Trennanordnung und die Mischung der Einzellösungen überprüft werden, ohne dass die Einzellösungen für die Bedienperson sichtbar eingefärbt werden müssten.

In einer weiteren bevorzugten Ausführungsform der erfindungsgemäßen Dialysemaschine erfolgt die Überprüfung der sachgerechten Öffnung der Trennanordnung auch durch Bestimmung eines Druckes oder einer Druckveränderung im Fluidsystem. Die Öffnung der Trennanordnung hat dabei einen Einfluß auf den Druck der Flüssigkeit im Mehrkammerbehälter bzw. auf den Druck, welchen die Flüssigkeit im Mehrkammerbehälter auf einen Druckmessbereich des Fluidsystems ausübt. Insbesondere erfolgt die Überprüfung dabei anhand des hydrostatischen Drucks der Flüssigkeit im Mehrkammerbehälter bzw. aus dem Mehrkammerbehälter.

Vorteilhafterweise wird dabei mindestens ein Ventilbereich des Fluidsystems geöffnet, so dass der Mehrkammerbehälter fluidisch mit dem Druckmessbereich des Fluidsystems in Verbindung steht. Dort bestimmt ein Drucksensor der Dialysemaschine den hydrostatischen Druck der Flüssigkeit und schließt aus diesem Druck bzw. aus einer Druckveränderung auf die Konfiguration des Mehrkammerbehälters.

Vorteilhafterweise wird der Mehrkammerbehälter dabei so an der Dialysemaschine angeordnet, dass der hydrostatische Druck am Ablauf bei geschlossener Trennanordnung sich von dem hydrostatischen Druck bei geöffneter Trennanordnung unterscheidet. Dies kann insbesondere dadurch erreicht werden, dass der Flüssigkeitspegel in der Kammer, an welcher der Ablauf angeordnet ist, bei geschlossener Trennanordnung niedriger oder höher ist als der Flüssigkeitspegel in der sich nach Öffnung der Trennanordnung bildenden Gesamtkammer.

Insbesondere kann dabei vorgesehen sein, dass der Flüssigkeitsspiegel in einer ersten Kammer bei geschlossener Trennanordnung höher angeordnet ist als der Flüssigkeitsspiegel in einer zweiten Kammer. Hierdurch verändert sich der hydrostatische Druck am Ablauf, wenn die Trennanordnung geöffnet wurde.

Insbesondere handelt es sich dabei bei dem Mehrkammerbehälter um einen Mehrkammerbeutel, welcher in einem ersten Randbereich an einen Ständer gehängt wird und bei welchem in einem zweiten, im wesentlichen gegenüberliegenden Randbereich des Beutels der Ablauf für die Flüssigkeit angeordnet ist. Die Trennnaht verläuft dabei vorteilhafterweise quer zwischen dem ersten und dem zweiten Bereich, so dass sich durch Öffnung der Trennnaht der hydrostatische Druck am Ausgang des Mehrkammerbeutels erhöht.

Die Dialysemaschine weist dabei vorteilhafterweise eine Verbindungsanordnung zur Verbindung des Mehrkammerbehälters mit der Dialysemaschine auf, insbesondere einen Haken zum Aufhängen eines Mehrkammerbeutels. Hierdurch kann ein definierter Höhenunterschied zwischen dem Mehrkammerbehälter und der Drucksensorik der Dialysemaschine sichergestellt werden. Die Verbindungsanordnung kann dabei z. B. an einem Gestell angeordnet sein, auf welchem die eigentliche Dialysemaschine angeordnet ist.

Die Bestimmung des Druckes erfolgt dabei vorteilhafterweise über einen Drucksensor zum Bestimmen des Druckes in einem Bereich des Fluidsystems, insbesondere in einer Kammer einer Kassette des Fluidsystems. Die Bestimmung des Druckes kann dabei direkt durch Ankoppeln eines Drucksensors an den Bereich des Fluidsystems, insbesondere an die Kammer, oder durch Bestimmen eines Druckes in einer Hydraulik eines Aktors, welcher an den Bereich des Fluidsystems, insbesondere an die Kammer der Kassette ankoppelt wird, erfolgen. Alternativ zum Ankoppeln des Drucksensors an eine Kammer einer Kassette kann der Drucksensor auch an das Schlauchset angekoppelt werden. Dies ist insbesondere dann der Fall, wenn keine Kassette eingesetzt wird, sondern das Dialysat z.B. über eine steuerbare Klemmvorrichtung und eine Schlauchpumpe in den Bauchraum des Patienten gepumpt wird. Die Bestimmung des hydrostatischen Drucks kann z.B. über eine ohnehin bei der Dialysemaschine vorgesehene Druckmesseinrichtung erfolgen, insbesondere über einen einer Pumpkammer zugeordneten Drucksensor. Die Dialysemaschine kann dabei zur Überprüfung der ordnungsgemäßen Öffnung der Trennanordnung die Pumpkammer mit dem Mehrkammerbeutel fluidisch in Verbindung bringen und durch Bestimmung des hydrostatischen Drucks in der Pumpkammer erkennen, ob die Trennanordnung sachgerecht geöffnet wurde.

Die Messung kann dabei dadurch erfolgen, dass ein Gleichgewicht zwischen dem Druck, der durch das Dialysat in der Pumpkammer entsteht, und dem Druck, welcher durch eine zum Bewegen einer Pumpmembran der Pumpkammer eingesetzen Hydraulikflüssigkeit erzeugt wird. Damit kann der Druck in der Pumpkammer durch einen Sensor auf der Hydraulikseite gemessen werden, da der Druck dort durch das Gleichgewicht dem Druck auf der Dialysatseite entspricht. Alternativ kann der Druck durch das Dialysat auch durch einen Sensor gemessen werden, welcher direkt an eine mit dem Mehrkammerbeutel fluidsich in Verbindung stehende Kammer angekoppelt wird.

In einer weiteren bevorzugten Ausführung der erfindungsgemäßen Dialysemaschine kann vorgesehen sein, dass die Überprüfung auch durch Bestimmung des Gewichts und/oder der Veränderung des Gewichts des Mehrkammerbeutels erfolgt. Insbesondere ist dafür eine Waageeinheit vorgesehen, über welche das Gewicht des Mehrkammerbehälters bestimmt werden kann. Die Öffnung der Trennanordnung hat dabei einen Einfluss auf die Flussgeschwindigkeit aus dem Mehrkammerbehälter, welche über das Gewicht des Mehrkammerbehälters bestimmt werden kann.

In weiterhin vorteilhafter Weise kann bei der erfindungsgemäßen Dialysemaschine vorgesehen sein, dass die Überprüfung der sachgerechten Öffnung der Trennanordnung auch durch Bestimmung der Flussgeschwindigkeit der aus dem Mehrkammerbehälter fließenden Flüssigkeit bestimmt wird. Die Bestimmung der Flussgeschwindigkeit kann dabei insbesondere über die oben beschriebene Bestimmung des Gewichts bzw. der Veränderung des Gewichts des Mehrkammerbehälters erfolgen. Alternativ oder zusätzlich kann die Bestimmung der Flussgeschwindigkeit auch über sonstige Bilanzierungsmittel der Dialysemaschine erfolgen.

In einer weiteren Ausführung der erfindungsgemäßen Dialysemaschine erfolgt die Überprüfung auch durch eine Ultraschall-Sensorik, insbesondere über eine Reflektions-Ultraschall-Sensorik. Die sachgerechte Öffnung der Trennanordnung kann dabei insbesondere durch das Erfassen einer Reflexionscharakteristik überprüft werden. Weiterhin kann vorgesehen sein, dass die Überprüfung anhand einer Füllstandserkennung mittels der Ultraschall-Sensorik erfolgt. Die Überprüfung kann dabei anhand eines absoluten Füllstands und/oder anhand einer Füllstandsänderung in dem Mehrkammerbehälter erfolgen.

Weiterhin kann vorgesehen sein, dass die Überprüfung anhand einer Änderung in der Reflexionscharakteristik, welche durch die Öffnung der Trennanordnung hervorgerufen wird, erfolgt. Damit kann insbesondere eine Öffnung der Trennanordnung überprüft werden, welche nach dem Ankoppeln des Mehrkammerbehälters an die Dialysemaschine erfolgt. Besonders geeignet ist dies zur Überprüfung einer selbsttätigen Öffnung der Trennanordnung durch die Dialysemaschine.

Die Ultraschall-Sensorik kann dabei bereits beim Ankoppeln des Mehrkammerbehälters an die Dialysemaschine an eine Kammer des Mehrkammerbehälters angekoppelt werden. In diesem Fall kann z.B. eine Änderung der Reflexionseigenschaften dieser Kammer bei der Öffnung der Trennanordnung zur Überprüfung herangezogen werden. Alternativ kann ein Ankoppeln der Ultraschall-Sensorik auch erst durch eine Änderung in der Form des Mehrkammerbeutels erfolgen, welche durch die Öffnung der Trennanordnung hervorgerufen wird. Entspricht in diesem Fall das Signal der Ultraschall-Sensorik dem einer angekoppelten Kammer, so kann hierüber auf die sachgerechte Öffnung geschlossen werden.

Die erfindungsgemäße Dialysemaschine umfasst bevorzugt Mittel zur selbsttätigen Öffnung der Trennanordnung. Hierdurch wird sichergestellt, dass die Trennanordnung vor Beginn der Behandlung sachgerecht geöffnet wurde, da dieses Öffnen von der Maschine selbst vorgenommen wird. Der Benutzer der Maschine muss die Trennanordnung daher nicht mehr selbst manuell öffnen.

Es ergeben sich besondere Vorteile, wenn solche Mittel zur selbsttätigen Öffnung der Trennanordnung mit der oben beschriebenen Vorrichtung zur automatischen Überprüfung der sachgerechten Öffnung der Trennanordnung des Mehrkammerbehälters kombiniert sind.

Vorteilhafterweise weisen die Mittel zur selbsttätigen Öffnung der Trennanordnung Mittel zum Druckaufbau in mindestens einer Kammer des Mehrkammerbehälters auf. Hierdurch kann die Trennanordnung sicher durch die Dialysemaschine geöffnet werden. Vorteilhafterweise erfolgt der Druckaufbau dabei über eine Leitung, über welche die Kammer des Mehrkammerbeutels mit der Dialysemaschine konnektiert wurde, insbesondere indem ein Fluid, d.h. ein Gas oder eine Flüssigkeit, in die Kammer gepumpt wird.

Weiterhin vorteilhafterweise erfolgt der Druckaufbau über das Einleiten von Pressluft oder das Pumpen von Flüssigkeit in die Kammer. Die Pressluft kann dabei der ohnehin vorhandenen Pressluft der Pneumatik der Dialysemaschine entnommen werden. Bei Einsatz von Pressluft wird diese vorteilhafterweise über einen Sterilfilter gereinigt. Wird der Druckanstieg über das Pumpen von Flüssigkeit in die Kammer erzeugt, so kann diese einer weiteren Kammer des Mehrkammerbehälters oder einer Kammer eines weiteren Mehrkammerbehälters entnommen werden.

Vorteilhafterweise umfasst die Steuerung eine Vorrichtung zur automatischen Überprüfung der sachgerechten Öffnung der Trennanordnung des Mehrkammerbehälters aufweist, welche die sachgerechte Öffnung der Trennanordnung auch durch die Mittel zur selbsttätigen Öffnung überprüft.

Insbesondere kann diese Überprüfung anhand eines Druckes und/oder eines Druckverlaufs und/oder einer Füllmenge erfolgen. Zum Öffnen der Trennanordnung ist dabei üblicherweise ein gewisser Überdruck in der mit Druck beaufschlagten Kammer notwendig. Die Vorrichtung zur Überwachung kann nun überprüfen, ob nach Überschreiten dieses Druckes ein Druckabfall durch die Öffnung der Trennanordnung stattfindet. Weiterhin kann die Vorrichtung zur Überwachung die Charakteristik des Druckanstiegs überprüfen, um zu erkennen, ob die Trennanordnung nicht bereits vorher geöffnet war. Hierbei kann die Vorrichtung ebenfalls überprüfen, ob der Druck nach Überschreiten des zur Öffnung notwendigen Druckes noch über einen zweiten, höheren Grenzwert ansteigt, ohne dass ein Druckabfall erfolgt. Wir der Druck durch das Pumpen von Flüssigkeit erzeugt, kann zudem die Menge an gepumpter Flüssigkeit überwacht und gegebenenfalls zusammen mit dem Druck ausgewertet werden.

Erfindungsgemäß kann also durch Beaufschlagen eines Fluides in einer ersten Kammer mit Druck, der durch die Dialysemaschine erzeugt wird, die Trennanordnung geöffnet werden. Im Moment des Öffnens der Trennanordnung kommt es durch das vergrößerte Volumen durch die zusätzliche Kammer zu einem meßbaren Druckabfall. Durch diesen Druckabfall kann verifiziert werden, dass die Trennanordnung geöffnet wurde. Hierbei ist es ebenfalls möglich zu detektieren, dass mehrere Trennanordnungen geöffnet wurden, da sich hierdurch ein bestimmter von der Art der Trennanordnungen abhängiger Druckverlauf ergibt.

Die vorliegende Erfindung umfasst weiterhin eine Dialysemaschine mit einem Fluidsystem mit einem Mehrkammerbehälter mit mindestens zwei durch eine mechanisch zu öffnende Trennanordnung getrennten Kammern mit Einzellösungen, wobei die Dialysemaschine und das Fluidsystem wie oben näher dargestellt aufgebaut sind.

Die vorliegende Erfindung umfasst weiterhin ein Verfahren zum Aufrüsten einer Dialysemaschine, insbesondere einer Peritonealdialysemaschine, wie sie oben beschrieben wurde, mit den Schritten:
- Ankoppeln eine Fluidsystems mit einem Mehrkammerbehälter mit mindestens zwei durch eine mechanisch zu öffnende Trennanordnung getrennten Kammern;
- Bestimmen einer Messgröße im Fluidsystem über einen Sensor der Dialysemaschine; und
- Überprüfen der sachgerechten Öffnung der Trennanordnung des Mehrkammerbehälters des an die Dialysemaschine angekoppelten Fluidsystems anhand der Messgröße,
wobei zur Überprüfung der sachgerechten Öffnung der Trennanordnung des Mehrkammerbehälters eine erste Kammer des Mehrkammerbehälters beheizt wird und eine zweite Kammer nicht beheizt wird, wobei die Überprüfung durch Bestimmung einer Temperatur oder einer Temperaturveränderung im Fluidsystem erfolgt.

Hierdurch kann - wie dies oben bereits dargestellt wurde - sichergestellt werden, dass die Trennanordnung sachgerecht geöffnet wurde und die korrekte Gesamtlösung für die Dialysebehandlung zum Einsatz kommt.

Insbesondere überprüft die Dialysemaschine die sachgerechte Öffnung der Trennanordnung automatisch.

Vorteilhafterweise umfasst das Verfahren dabei das Auslösen eines Alarms und/oder die Verhinderung eines Starts der Behandlung, wenn die Messgröße nicht einer bei einer sachgerechten Öffnung der Trennanordnung erwarteten Messgröße entspricht, insbesondere wenn die Messgröße außerhalb eines vorgegebene Bereichs oder unter- oder oberhalb eines Grenzwertes liegt oder der Verlauf der Messgröße nicht einem erwarteten Verlauf entspricht.

Das Verfahren umfasst bevorzugt den Schritt: selbsttätiges Öffnen der Trennanordnung durch die Dialysemaschine.

Vorteilhafterweise erfolgt das selbsttätige Öffnen der Trennanordnung dabei so, wie dies oben hinsichtlich der Dialysemaschine beschrieben wurden. Insbesondere handelt es sich bei dem Verfahren dabei um ein Verfahren zum Aufrüsten einer oben beschriebenen Dialysemaschine.

Vorteilhafterweise ist neben dem selbsttätigen Öffnen dabei eine automatische Überprüfung vorgesehen, wie sie ebenfalls oben beschrieben wurde.

Die vorliegende Erfindung wird nun anhand von Ausführungsbeispielen sowie Zeichnungen näher dargestellt.
- Fig. 1: drei Diagramme, welche typische Verläufe einer automatischen Peritonealdialysebehandlung zeigen,
- Fig. 2: eine Prinzipdarstellung eines Peritonealdialysesystems,
- Fig. 3: eine Prinzipdarstellung der Aufteilung des Peritonealdialysesystems in eine Dialysemaschine und ein Fluidsystem,
- Fig. 4: ein erstes Ausführungsbeispiel einer Kassette,
- Fig. 5: ein zweites Ausführungsbeispiel einer Kassette,
- Fig. 6: eine perspektivische Ansicht eines ersten Ausführungsbeispiels einer Dialysemaschine,
- Fig. 7: ein Flussdiagramm eines ersten Ausführungsbeispiels eines Peritonealdialysesystems,
- Fig. 8: eine perspektivische Ansicht eines zweiten Ausführungsbeispiels einer Dialysemaschine,
- Fig. 9: ein Flussdiagramm eines zweiten Ausführungsbeispiels eines Peritonealdialysesystems,
- Fig. 10: das Ankoppeln der Kassette beim zweiten Ausführungsbeispiels eines Peritonealdialysesystems,
- Fig. 11: ein erstes Ausführungsbeispiels eines Pumpaktors,
- Fig. 12: das Ankoppeln eines Pumpbereichs der Kassette an einen Pumpaktor,
- Fig. 13: eine Prinzipdarstellung des Aufbaus eines Ausführungsbeispiels einer Steuerung,
- Fig. 14: ein Ausführungsbeispiel eines Mehrkammerbehälters, welcher an ein Ausführungsbeispiel einer erfindungsgemäßen Dialysemaschine ankoppelbar ist,
- Fig. 15: ein Ausführungsbeispiel einer Heizung gemäß einem Ausführungsbeispiel der vorliegenden Erfindung,
- Fig. 16: ein Ausführungsbeispiel eines optischen Sensors gemäß der vorliegenden Erfindung,
- Fig. 17: ein erstes Ausführungsbeispiel einer Ultraschall-Sensorik gemäß der vorliegenden Erfindung, und
- Fig. 18: ein zweites Ausführungsbeispiel einer Ultraschall-Sensorik gemäß der vorliegenden Erfindung.

Im folgenden soll die Funktion einer Dialysemaschine, bei welcher die vorliegende Erfindung zum Einsatz kommt, zunächst allgemein beschrieben werden. Bei der Dialysemaschine handelt es sich dabei im Ausführungsbeispiel um eine Peritonealdialysemaschine. Die unten beschriebenen Komponenten können jedoch in gleicher oder ähnlicher Weise auch für eine Hämodialysemaschine eingesetzt werden.

Die Peritonealdialyse ist eine Variante der künstlichen Blutwäsche, bei welcher das gut durchblutete Bauchfell (Peritoneum) des Patienten als körpereigene Filtermembran verwendet wird. Hierfür wird über einen Katheter Dialysat in die Bauchhöhle eingeführt. Nach dem Prinzip der Osmose diffundieren nun Harnbestandteile des Blutes durch das Bauchfell in das in der Bauchhöhle befindliche Dialysat. Nach einer gewissen Verweilzeit wird das Dialysat mit den Harnbestandteilen wieder aus der Bauchhöhle ausgelassen.

Bei der automatischen Peritonealdialyse steuert und überwacht eine Dialysemaschine die Einführung des frischen Dialysats in die Bauchhöhle und das Auslassen des verbrauchten Dialysats. Eine solche Dialysemaschine, auch Cycler genannt, befüllt und entleert die Bauchhöhle dabei üblicherweise mehrmals über Nacht, d.h. während der Patient schläft.

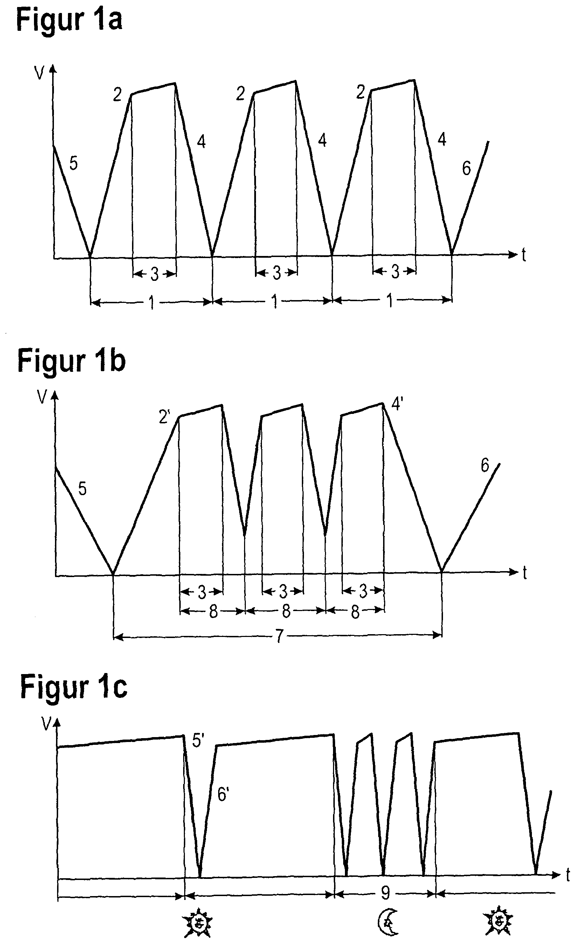

In Figuren 1a bis 1c sind drei unterschiedliche Verfahrensabläufe gezeigt, wie sie von einer Dialysemaschine durchgeführt werden. Einer oder mehrerer dieser Verfahrensabläufe ist dabei üblicherweise in der Steuerung der Dialysemaschine hinterlegt. Dabei ist es üblicherweise möglich, die abgespeicherten Verfahrensabläufe an den Patienten anzupassen.

Bei den Figuren 1a bis 1c ist jeweils die in der Bauchhöhle des Patienten befindliche Dialysatmenge V über die Zeit t aufgezeichnet. Figur 1a zeigt dabei den Verlauf einer normalen automatischen Peritonealdialysebehandlung über Nacht. Zu Beginn der Behandlung erfolgt dabei zunächst ein initialer Auslauf 5, durch welchen Dialysat, das über den Tag in der Bauchhöhle des Patienten belassen worden war, entnommen wird. Daraufhin folgen mehrere Behandlungszyklen 1, in Figur 1a drei aufeinander folgende Behandlungszyklen 1. Jeder Behandlungszyklus besteht dabei aus einer Einlaufphase 2, einer Verweilphase 3 und einer Auslaufphase 4. Während der Einlaufphase 2 wird dabei ein gewisses Volumen an frischer Dialyseflüssigkeit in die Bauchhöhle des Patienten eingelassen. Die maximal zulässige Dialysatmenge beträgt dabei je nach Patienten zwischen ca. 1,5 und 3 I. Das frische Dialysat verbleibt nun über eine gewisse Verweilzeit 3 in der Bauchhöhle. Typischerweise dauert die Verweilphase dabei einige Stunden. Daraufhin wird das nun verbrauchte Dialysat in der Auslaufphase 4 wieder aus der Bauchhöhle herausgelassen. Daraufhin startet ein neuer Behandlungszyklus. Die Behandlung wird mit einem letzten Einlauf 6 abgeschlossen, durch welchen eine gewissen Menge an frischem Dialysat in die Bauchhöhle des Patienten eingeführt wird. Diese verbleibt dann über den Tag in der Bauchhöhle des Patienten.

Die einzelnen Behandlungszyklen 1, welche über Nacht erfolgen, werden dabei automatisch von der Steuerung der Dialysemaschine angesteuert. Der initiale Auslauf und der letzte Einlauf können ebenfalls automatisch von der Dialysemaschine angesteuert werden. Alternativ werden diese manuell von einer Bedienperson oder vom Patienten aktiviert.

In Figur 1b ist eine sogenannte Tidalbehandlung gezeigt. Auch diese beginnt mit einem initialen Auslauf 5 und endet mit einem letzten Einlauf 6. Weiterhin ist ein Basiszyklus 7 vorgesehen, welcher sich in mehrere Tidalzyklen 8 aufteilt. Dabei ist zunächst eine Basiseinlaufphase 2' vorgesehen. Nach der Verweilphase 3 wird jedoch nicht mehr das komplette Dialysatvolumen aus der Bauchhöhle entnommen, sondern nur eine gewisse Teilmenge des in der Bauchhöhle befindlichen Dialysats. Dieses wird dann durch ein entsprechendes Volumen an frischem Dialysat ersetzt. Nach einem erneuten Verweilzyklus kann eine weitere Tidalentnahme erfolgen, bei welcher nicht das gesamte im Bauchraum befindliche Dialysat entfernt wird. Zum Ende des Basiszyklus 7 erfolgt eine Basisauslaufphase 4', bei welcher nun das gesamte Dialysat entnommen wird. In Figur 1b ist dabei lediglich ein Basiszyklus 1 dargestellt. Alternativ können jedoch auch mehrere Basiszyklen vorgesehen sein.

In Figur 1c ist der Verlauf einer Peritonealdialysebehandlung mit einer sogenannten PD-Plus-Behandlung gezeigt. Dabei erfolgt während der Nacht 9 eine übliche Peritonealdialysebehandlung, welche z. B. gemäß den Figuren 1a oder 1b durchgeführt werden kann. Weiterhin ist jedoch während des Tages eine zusätzliche PD-Plus-Behandlung vorgesehen, bei welcher in einer Auslaufphase 5' das verbrauchte Dialysat entnommen und in einer Einlaufphase 6' durch frisches Dialysat ersetzt wird. Bei der PD-Plus-Behandlung wird damit eine normale nächtliche Peritonealdialysebehandlung mit einem oder mehreren zusätzlichen Behandlungszyklen während des Tages kombiniert. Der Verlauf der nächtlichen Behandlung wird dabei wie üblich automatisch durch die Dialysemaschine durchgeführt. Die Behandlungszyklen während des Tages werden ebenfalls über die Maschine durchgeführt und überwacht.

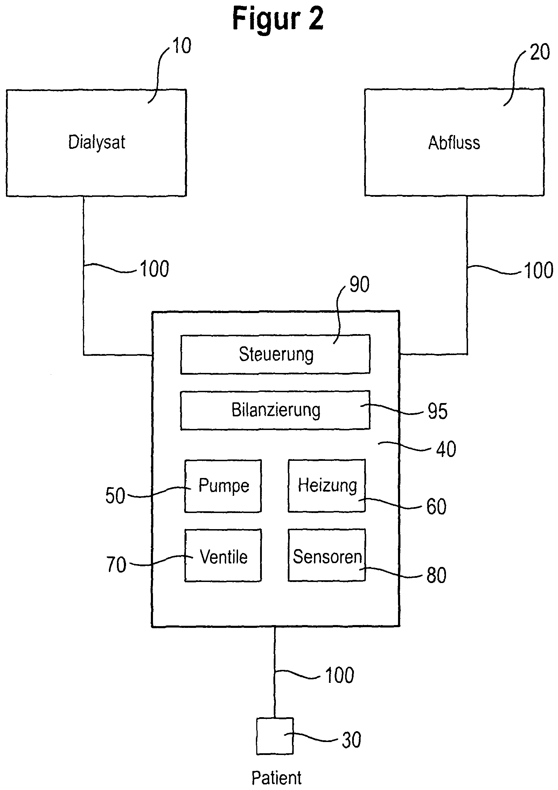

In Figur 2 ist nun der Aufbau eines typischen Peritonealdialysesystems schematisch dargestellt. Das Peritonealdialysesystem umfasst dabei einen Behälter 10 mit frischem Dialysat und einen Abfluß 20 für gebrauchtes Dialysat. Weiterhin ist ein Konnektor 30 vorgesehen, welcher an einen Katheter des Patienten angeschlossen werden kann, um entweder frisches Dialysat in die Bauchhöhle des Patienten einzuführen oder gebrauchtes Dialysat aus der Bauchhöhle abzuführen. Der Behälter 10 mit frischem Dialysat, der Abfluß 20 für gebrauchtes Dialysat und der Konnektor 30 zum Patienten sind dabei über Fluidwege 100 miteinander verbunden und bilden zusammen mit diesen das Fluidsystem des Peritonealdialysesystems.

Zur Durchführung der Peritonealdialysebehandlung ist eine Dialysemaschine 40, auch Cycler genannt, vorgesehen. Die Dialysemaschine 40 umfasst dabei folgende Hauptkomponenten:
- Eine Pumpe 50, welche zum Transport der Flüssigkeiten eingesetzt wird. Die Pumpe 50 befördert dabei das frische Dialysat vom Behälter 10 zum Konnektor 30. Weiterhin kann die Pumpe 50 das verbrauchte Dialysat vom Konnektor 30 zum Abfluß 20 transportieren.
- Ventile 70, welche zur Steuerung der Flüssigkeitsströme eingesetzt werden. Die Ventile 70 öffnen und schließen die Fluidwege 100, um so die entsprechenden Fluidverbindungen zwischen dem Behälter 10, dem Konnektor 30 und dem Abfluß 20 herzustellen.
- Eine Heizung 60, welche das frische Dialysat auf eine Temperatur von ca. 37°C bringt, bevor dieses dem Patienten zugeführt wird. Da bei der Peritonealdialyse relativ große Mengen an Dialysat direkt in die Bauchhöhle des Patienten eingeführt werden, ist die Heizung 60 nötig, um den Patienten nicht zu unterkühlen und um ein unangenehmes Gefühl durch zu kaltes Dialysat zu vermeiden.
- Sensoren 80, über welche der ordnungsgemäße Ablauf der Behandlung überwacht und/oder gesteuert werden kann. Insbesondere können dabei Temperatursensoren zum Einsatz kommen. Weiterhin können gegebenenfalls Drucksensoren zum Einsatz kommen.

Alle Komponenten der Dialysemaschine 40 werden dabei über eine Steuerung 90 angesteuert. Die Steuerung 90 steuert dabei insbesondere die Pumpe 50, die Heizung 60 und die Ventile 70 auf Grundlage der Daten der Sensoren 80 an. Die Steuerung 90 sorgt dabei für den automatischen Ablauf der Peritonealdialyse. Als wichtige Komponente umfasst die Steuerung 90 dabei eine Bilanzierung 95, welche die dem Patienten zugegebenen und entnommenen Flüssigkeitsmengen bilanziert. Die Bilanzierung verhindert dabei, dass dem Patienten zuviel Flüssigkeit zugegeben oder zuviel Flüssigkeit entnommen wird.

Die Bilanzierung 95 kann dabei allein auf Grundlage der Ansteuerdaten und/oder der Sensordaten für die Pumpe 50 erfolgen. Alternativ kann die Bilanzierung auch über separat vorgesehene Bilanzierkammern erfolgen. Ebenso ist es möglich, zur Bilanzierung eine Waage einzusetzen. Eine solche Waage wiegt beispielsweise das Gewicht des Behälters 10 mit frischem Dialysat und/oder eines Behälters 20 mit gebrauchtem Dialysat.

Da bei der Peritonealdialyse das Dialysat dem Patienten direkt in die Bauchhöhle verabreicht wird, ist auf äußerste Sterilität zu achten. Daher werden die Fluidwege bzw. das Fluidsystem, welches mit dem frischen und/oder dem gebrauchten Dialysat in Kontakt kommt, üblicherweise als Einwegteil ausgeführt. Insbesondere werden die Fluidwege bzw. das Fluidsystem dabei als Kunststoffteile ausgeführt. Diese können so in einer sterilen Umverpackung angeliefert und erst kurz vor der Behandlung ausgepackt werden.

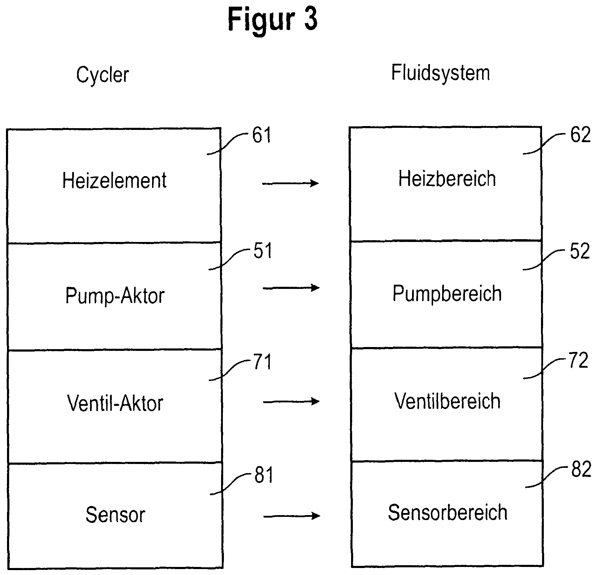

Um dennoch eine Steuerung der Peritonealdialyse durch die Dialysemaschine 40 zu ermöglichen, muß das Fluidsystem an die Dialysemaschine 40 angekoppelt werden. In Figur 3 ist dabei schematisch dargestellt, wie einzelne Elemente der Dialysemaschine 40 an entsprechende Bereiche des Fluidsystems angekoppelt werden.

Die Dialysemaschine 40 weist dabei ein Heizelement 61 auf. Dieses muss an einen entsprechenden Heizbereich 62 des Fluidsytems angekoppelt werden. Die Ankopplung ermöglicht dabei die Übertragung von Wärmeenergie von dem Heizelement 61 an das im Heizbereich 62 befindliche Dialysat.

Die Dialysemaschine 40 weist weiterhin einen oder mehrere Pumpaktoren 51 auf, welche mit einem Pumpbereich 52 des Fluidsystems gekoppelt werden. Die Pumpaktoren 51 erzeugen dabei eine Pumpkraft, welche auf den Pumpbereich 52 übertragen wird. Hierdurch kann die im Pumpbereich 52 befindliche Flüssigkeit entlang der Fluidwege bewegt werden.

Weiterhin weist die Dialysemaschine einen oder mehrere Ventilaktoren 71 auf. Diese erzeugen eine Schließbewegung, welche auf entsprechende Ventilbereiche 72 der Fluidwege übertragen wird. Hierdurch können die Ventilbereiche 72 der Fluidwege entsprechend geschlossen bzw. geöffnet werden.

Weiterhin weist die Dialysemaschine einen oder mehrere Sensoren 81 auf. Diese werden an einen entsprechenden Sensorbereiche 82 des Fluidsystems angekoppelt. Hierdurch können die Sensoren 81 gewisse Eigenschaften des Dialysats messen. Insbesondere kann hierdurch die Temperatur des Dialysats gemessen werden. Weiterhin kann vorgesehen sein, dass der Druck in dem Fluidsystem bestimmt wird.

Selbstverständlich weist die Dialysemaschine gegebenenfalls noch weitere Aktoren und/oder Sensoren auf, welche nicht mit den Fluidwegen gekoppelt werden müssen.

Die einzelnen Komponenten eines Peritonealdialysesystems sollen nun im folgenden näher anhand von Ausführungsbeispielen dargestellt werden.

### 1. Fluidsystem

### 1.1 Dialysatbehälter

Frisches Dialysat wird üblicherweise in Kunststoffbeuteln zur Verfügung gestellt. Solche Kunststoffbeutel weisen üblicherweise zwei Lagen von Kunststofffolie auf, welche in einem Randbereich miteinander verschweißt sind und so einen Behälter bilden, welcher mit frischem Dialysat befüllt ist. An diesen Behälter ist üblicherweise ein Schlauchelement angeschweißt, durch welches das Dialysat aus dem Beutel entnommen werden kann. An dem Schlauchelement ist üblicherweise ein Konnektor angeordnet, über welchen der Dialysatbehälter mit den übrigen Fluidwegen verbunden werden kann. Weiterhin weist der Beutel üblicherweise an der dem Schlauch gegenüberliegenden Seite eine Aussparung oder Öse auf, über welche der Beutel an einen Haken aufgehängt werden kann. Hierdurch kann sichergestellt werden, dass das Dialysat problemlos aus dem Beutel abfließt.

Das Dialysat besteht üblicherweise aus einem Puffer, einem Osmotikum und Elektrolyten. Als Puffer kann dabei z. B. Bicarbonat eingesetzt werden. Als Osmotikum wird üblicherweise Glucose eingesetzt. Alternativ können auch Glucosepolymere oder Glucosepolymerderivate eingesetzt werden. Die Elektrolyte umfassen üblicherweise Kalzium und Natrium.

Das Dialysat kann dabei hitzesterilisiert werden. Dies erfolgt vorteilhafterweise, nachdem das Dialysat in den Beutel gefüllt wurde. Hierdurch werden sowohl das Dialysat als auch der Beutel hitzesterilisiert. Dabei wird der gefüllte Beutel üblicherweise zunächst in eine Umverpackung verpackt, woraufhin das gesamte System sterilisiert wird.

Da die fertige Dialysatlösung je nach Inhaltsstoffen oft nicht hitzesterilisiert werden kann oder nicht lange gelagert werden kann, kann vorgesehen sein, einzelne Komponenten des Dialysats getrennt zu lagern und erst kurz vor der Behandlung zusammenzuführen. Eine erste Einzellösung umfasst dabei üblicherweise den Puffer, während eine zweite Einzellösung Glucose und Elektrolyte umfasst. Gegebenenfalls können auch mehr als zwei Einzellösungen und damit mehr als zwei Bereiche in einem Beutel vorgesehen sein. Dabei kann ein Mehrfachkammerbeutel, insbesondere ein Doppelkammerbeutel vorgesehen sein, welcher mehrere getrennte Bereiche zur Lagerung der Einzellösungen aufweist. Diese Bereiche sind durch ein Verbindungselement getrennt, welches mechanisch geöffnet werden kann, um die Einzelflüssigkeiten miteinander zu vermischen. Insbesondere kann dabei eine sogenannte Peel-Naht zwischen den beiden Bereichen des Beutels vorgesehen sein, welche sich bei Anwendung eines bestimmten Druckes auf mindestens einen der Bereiche des Beutels öffnet.

Da während einer nächtlichen Peritonealdialysebehandlung relativ große Mengen an Dialysat verbraucht werden, werden üblicherweise mehrere Dialysatbehälter parallel eingesetzt. Diese werden über entsprechende Konnektoren mit den Fluidwegen verbunden und können durch eine entsprechende Schaltung der Ventilen zum Befüllen des Patienten herangezogen werden.

### 1.2 Abfluß

Zum Entsorgen der verbrauchten Dialysierflüssigkeit kann diese entweder sofort in die Kanalisation abgeführt oder zunächst in einem Abflußbehälter gesammelt werden. Als Abflußbehälter wird dabei üblicherweise ebenfalls ein Beutel eingesetzt. Dieser ist vor Beginn der Behandlung leer und kann so das verbrauchte Dialysat aufnehmen. Der Beutel kann dann nach Beendigung der Behandlung entsprechend entsorgt werden.

### 1.3 Kassette

Wie bereits eingangs beschrieben, weist das Fluidsystem eine Mehrzahl von Bereichen auf, in welchen die Dialysemaschine auf das Fluidsystem einwirken muss. Hierfür muss das Fluidsystem an die Dialysemaschine angekoppelt werden.

Um das Ankoppeln der Fluidwege an die Dialysemaschine und die Einwirkung der entsprechenden Elemente der Dialysemaschine auf die Fluidwege zu vereinfachen, werden Kassetten eingesetzt. In einer solchen Kassette sind mehrere Bereiche, in welchen die Dialysemaschine auf die Fluidwege einwirkt, gemeinsam angeordnet. Hierfür weist eine Kassette üblicherweise ein Hartteil aus Kunststoff auf, in welches zu einer Seite hin offene Kammern als Fluidwege eingebracht sind. Diese Kammern werden von einer flexiblen Kunststofffolie bedeckt, welche für die Ankopplung an die Dialysemaschine sorgt. Die flexible Kunststofffolie ist dabei üblicherweise in einem Randbereich mit dem Hartteil verschweißt. Die Kassette wird mit einer Ankoppelfläche der Dialysemaschine verpresst, so dass die Aktoren und/oder Sensoren der Dialysemaschine mit entsprechenden Bereichen der Kassette in Kontakt kommen.

Die Kassette weist weiterhin Anschlüsse zum Anschluss des Dialysatbehälters 10, des Konnektors 30 sowie des Ablaufs 20 auf.

Eine Kassette umfasst dabei üblicherweise zumindest einen Pumpbereich und einen oder mehrere Ventilbereiche. Über die Kassette kann so der Flüssigkeitstransport durch das Fluidsystem gesteuert werden. Weiterhin kann die Kassette Sensorbereiche aufweisen, welche eine einfache Ankopplung von Sensoren der Dialysemaschine an das Fluidsystem ermöglichen. Gegebenenfalls kann die Kassette weiterhin einen oder mehrere Heizbereiche aufweisen, welche an entsprechende Heizelemente der Dialysemaschine ankoppelbar sind.

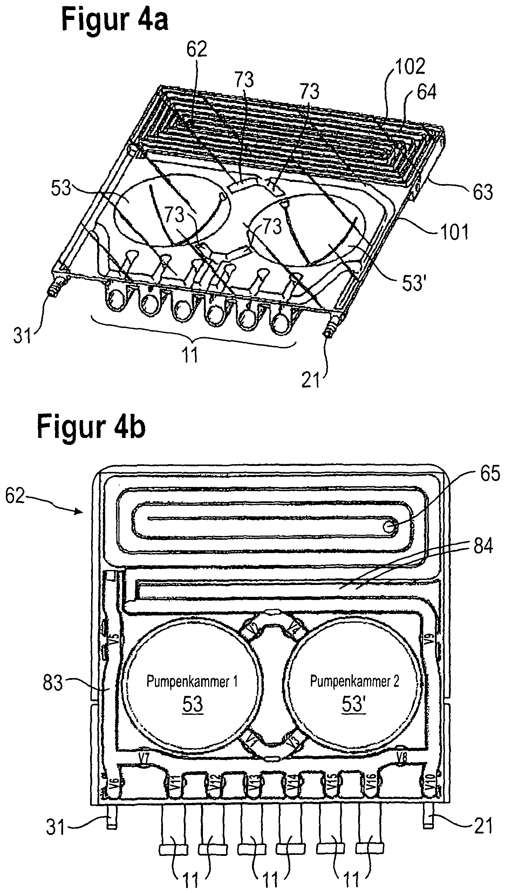

In Figuren 4a und 4b ist ein erstes Ausführungsbeispiel einer Kassette dargestellt. Diese weist ein Hartteil 101 aus Kunststoff auf, in welchem die Fluidwege und Ankopplungsbereiche als entsprechende Aussparungen, Kammern und Kanäle eingebracht sind. Das Hartteil kann dabei z. B. als Spritzgussteil oder als Tiefziehteil gefertigt werden. Die Ankoppelebene des Hartteils 101 wird von einer flexiblen Folie 102 bedeckt, welche in einem Randbereich mit dem Hartteil verschweißt ist. Durch das Verpressen der Kassette mit einer Ankoppelfläche der Dialysemaschine wird die flexible Folie 102 mit dem Hartteil verpresst. Durch das Verpressen der flexiblen Folie mit den Stegbereichen des Hartteils werden die Fluidwege innerhalb der Kassette fluiddicht voneinander getrennt.

Die Kassette weist Anschlüsse zum Anschluss der Kassette an die übrigen Fluidwege auf. Zum einen ist ein Anschluss 21 zum Anschluss an den Abfluß 20 sowie ein Anschluss 31 zum Anschluss an den Konnektor 30 vorgesehen. An diesen Anschlüssen können entsprechende Schlauchelemente vorgesehen sein, welche in Figur 4a nicht dargestellt sind. Weiterhin weist die Kassette eine Mehrzahl von Anschlüssen 11 zum Anschluss von Dialysatbehältern 10 auf. Die Anschlüsse 11 sind dabei im ersten Ausführungsbeispiel als Konnektoren ausgeführt, an welche entsprechende Konnektorelemente angeschlossen werden können.

Die Anschlüsse stehen jeweils mit Fluidwegen innerhalb der Kassette in Verbindung. In diesen Fluidwegen sind Ventilbereiche vorgesehen. In diesen Ventilbereichen kann die flexible Folie 102 über maschinenseitige Ventilaktoren so in das Hartteil 101 gedrückt werden, dass der entsprechende Fluidweg versperrt ist. Die Kassette weist dabei zunächst einmal für jeden Anschluss ein entsprechendes Ventil auf, über welches dieser Anschluss geöffnet bzw. geschlossen werden kann. Dem Anschluss 21 für den Abfluß 20 ist dabei das Ventil V10 zugeordnet, dem Anschluss 31 für den Patientenkonnektor 30 das Ventil V6. Den Anschlüssen 11 für die Dialysatbehälter 10 sind die Ventile V11 bis V16 zugeordnet.

Weiterhin sind in der Kassette Pumpenkammern 53 und 53' vorgesehen, welche durch entsprechende Pumpaktoren der Dialysemaschine betätigt werden können. Bei den Pumpenkammern 53 und 53' handelt es sich dabei um konkave Aussparungen in dem Hartteil 101, welche von der flexiblen Folie 102 bedeckt werden. Durch Pumpaktoren der Dialysemaschine kann die Folie nun in die Pumpenkammern 53 und 53' hineingedrückt bzw. wieder aus diesen Pumpenkammern herausgezogen werden. Hierdurch kann, im Zusammenspiel mit den Ventilen V1 bis V4, welche die Zugänge und Abläufe der Pumpenkammern 53 und 53' schalten und in Fig. 4a mit dem Bezugszeichen 73 bezeichnet wurden, ein Pumpstrom durch die Kassette erzeugt werden. Die Pumpkammern sind dabei über entsprechende Ventilschaltungen mit allen Anschlüssen der Kassette verbindbar.

Weiterhin ist ein Heizbereich 62 in die Kassette integriert. In diesem Bereich wird die Kassette mit Heizelementen der Dialysemaschine in Kontakt gebracht, welche das durch diesen Bereich der Kassette fließende Dialysat erwärmen. Der Heizbereich 62 weist dabei einen Kanal für das Dialysat auf, welcher sich spiralförmig über den Heizbereich 62 erstreckt. Der Kanal wird dabei durch Stege 64 des Hartteils gebildet, welche von der flexiblen Folie 102 abgedeckt sind.

Der Heizbereich 62 ist dabei auf beiden Seiten der Kassette vorgesehen. Hierfür ist auch auf der Unterseite 63 der Kassette im Heizbereich eine flexible Folie am Hartteil angeordnet. Die flexible Folie ist dabei ebenfalls in einem Randbereich mit dem Hartteil verschweißt. Auf der Unterseite ist ebenfalls ein Kanal angeordnet, durch welchen das Dialysat fließt. Die Kanäle auf der Unterseite und der Oberseite werden dabei durch eine mittlere Platte des Hartteils gebildet, welche die Oberseite von der Unterseite trennt, und auf welcher nach unten und nach oben Stege vorgesehen sind, welche die Kanalwandungen bilden. Dabei fließt das Dialysat zunächst spiralförmig auf der Oberseite bis zum Durchbruch 65 durch die mittlere Platte, von wo aus das Dialysat auf der Unterseite durch den entsprechenden Kanal zurückfließt. Durch den auf der Ober- und der Unterseite vorgesehenen Heizbereich kann die Heizfläche, welche zum Aufheizen der Flüssigkeit zur Verfügung steht, entsprechend vergrößert werden. Selbstverständlich ist aber auch eine Ausführungsform der Kassette möglich, bei welcher nur auf einer Seite der Kassette ein Heizbereich angeordnet ist.

Weiterhin sind Ausführungsformen der Kassette möglich, bei welcher ein Heizelement in die Kassette integriert ist. Insbesondere kann dabei ein elektrisches Heizelement wie z.B. Heizwendel in das Hartteil der Kassette eingegossen sein. Hierdurch kann auf ein maschienenseitiges Heizelement verzichtet werden und die Durchflussheizung in die Kassette integriert werden. Dabei sind an der Kassette elektrische Kontakte zur Konnektierung des elektrischen Heizelements angeordnet.

Die Kassette weist weiterhin Sensorbereiche 83 und 84 auf, durch welche Temperatursensoren der Dialysemaschine an die Kassette gekoppelt werden können. Die Temperatursensoren liegen dabei auf der flexiblen Folie 102 auf und können so die Temperatur der durch den darunter liegenden Kanal fließenden Flüssigkeit messen. Dabei sind am Eingang des Heizbereiches zwei Temperatursensoren 84 vorgesehen. Am patientenseitigen Ausgang ist ein Temperatursensor 83 vorgesehen, über welchen die Temperatur des zum Patienten gepumpten Dialysats gemessen werden kann.

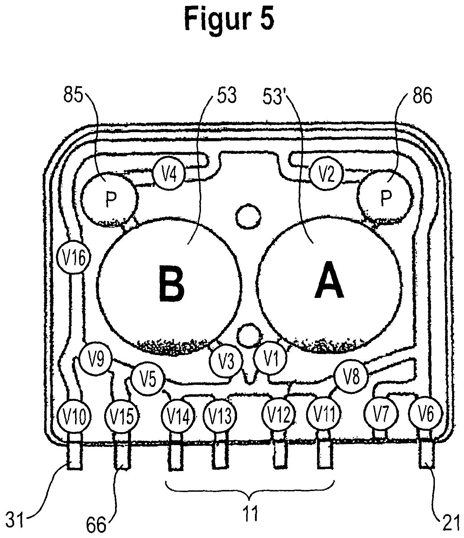

In Figur 5 ist ein zweites Ausführungsbeispiel für eine Kassette gezeigt. Die Kassette entspricht dabei in ihrer Ausführung im wesentlichen dem ersten Ausführungsbeispiel, umfasst jedoch keinen Heizbereich. Bei Einsatz dieser Kassette erfolgt die Heizung daher nicht wie beim ersten Ausführungsbeispiel gezeigt über einen in die Kassette integrierten Heizbereich, sondern z. B. über einen Heizbeutel, welcher auf eine Heizplatte der Dialysemaschine gelegt wird.

Das in Figur 5 gezeigte zweite Ausführungsbeispiel einer Kassette weist wiederum Fluidwege auf, welche über Ventilbereiche, die hier ebenfalls von V1 bis V16 durchnumeriert sind, geöffnet und geschlossen werden können. Weiterhin weist die Kassette Anschlüsse zum Anschluss an weitere Komponenten des Fluidsystems auf. Dabei ist wiederum der Anschluss 21 zum Anschluss an den Abfluß 20, sowie der Anschluss 31 zum Anschluss an den Konnektor 30 zum Patienten vorgesehen. Weiterhin sind Anschlüsse 11 zum Anschluss von Dialysatbehältern 10 vorgesehen.

Im Unterschied zum ersten Ausführungsbeispiel weist die im zweiten Ausführungsbeispiel gezeigte Kassette einen weiteren Anschluss 66 zum Anschluss eines Heizbeutels auf. Zum Erwärmen der Flüssigkeit aus den Dialysatbehältern 10 kann die Flüssigkeit dabei über den Anschluss 66 in einen Heizbeutel gepumpt werden. Dieser Heizbeutel liegt auf einem Heizelement auf, so dass die im Heizbeutel befindliche Flüssigkeit erwärmt werden kann. Daraufhin wird die Flüssigkeit aus dem Heizbeutel zum Patienten gepumpt.

Die Pumpkammer 53 und 53' und die Ventile V1 bis V4 entsprechen in Aufbau und Funktion den entsprechenden Komponenten im ersten Ausführungsbeispiel.

Im Unterschied zum ersten Ausführungsbeispiel weist die Kassette im zweiten Ausführungsbeispiel keinen Sensorbereich zum Anschluss eines Temperatursensors auf. Dieser ist vielmehr im Bereich der Heizelemente angeordnet. Die Kassette weist jedoch Messbereiche 85 und 86 zum Messen des Drucks in den Pumpekammern 53 und 53' auf. Die Messbereiche 85 und 86 sind dabei Kammern, welche mit dem Pumpkammern fluidisch in Verbindung stehen und ebenfalls von der flexiblen Folie abgedeckt werden. An die Messbereiche können geräteseitige Drucksensoren angekoppelt werden, welche den Druck in den Messkammern 85 und 86 und damit in den Pumpkammern 53 und 53' messen.

Die Verbindung der Anschlüsse 11, 21, 31 und 66 der Kassette mit den weiteren Komponenten des Fluidsystems erfolgt im zweiten Ausführungsbeispiel über Schlauchverbindungen. An diesen Schlauchverbindungen sind gegebenenfalls Konnektoren angeordnet.

### 1.3 Schläuche

Die Verbindung zwischen den einzelnen Behältern des Systems, der Kassette und dem Patientenkonnektor erfolgt üblicherweise über Schlauchverbindungen. Da es sich jeweils um Einwegartikel handelt, sind die Schläuche dabei üblicherweise zumindest auf einer Seite bereits mit einem weiteren Element fest verbunden. Z. B. können Schläuche bereits an einem oder mehreren der Anschlüsse der Kassette vorgesehen sein. Ebenfalls können Schläuche bereits fest mit Beuteln in Verbindung stehen.

### 1.4 Verbindungen

Das Fluidsystem ist üblicherweise in mehrere Teile aufgeteilt und jeweils steril verpackt. Diese Teile müssen für die Behandlung zunächst miteinander verbunden werden. Insbesondere sind dabei üblicherweise die Kassette sowie der oder die Dialysatbeutel getrennt voneinander verpackt.

Die Verbindungen zwischen den einzelnen Elementen des Fluidsystems erfolgt üblicherweise über Konnektoren. Die Konnektoren sind dabei so gestaltet, dass sie eine sterile Verbindung zwischen den einzelnen Komponenten ermöglichen. Dies erfolgt z. B. über entsprechende Schutzfolien, welche beim Schließen des Konnektors automatisch geöffnet werden.

Die Verbindung der einzelnen Komponenten kann dabei manuell durch eine Bedienperson bzw. den Patienten selbst erfolgen. Alternativ kann vorgesehen sein, dass die Verbindung der einzelnen Komponenten durch die Dialysemaschine erfolgt.

Hierzu können z. B. die entsprechenden Konnektoren in eine Konnektoraufnahme der Dialysemaschine eingelegt und automatisch durch die Dialysemaschine zusammengeführt werden.

Weiterhin kann eine elektronische Steuerung vorgesehen sein, welche überwacht, dass die richtigen Komponenten des Systems miteinander verbunden werden. Hierfür können an den Konnektoren Identifikationsmittel wie z. B. Barcodes oder RFIDs vorgesehen sein, welche die Komponenten identifizieren. Die Dialysemaschine umfasst dabei eine Identifikationsmittel-Erfassungseinheit wie z. B. einen Barcodeleser oder eine RFID-Erfassungseinheit, welche die Identifikationsmittel auf den Konnektoren erfasst. Hierdurch kann die Steuerung der Peritonealdialyse erkennen, ob die korrekten Konnektoren eingelegt wurden.

Eine solche Überprüfung der korrekten Zusammenstellung des Fluidsystems kann dabei insbesondere mit einer automatischen Konnektion der Konnektoren kombiniert sein. Das System überprüft so zunächst, ob die richtigen Konnektoren in die Konnektorenaufnahmen eingelegt wurden. Die Verbindung zwischen den Konnektoren wird durch die Dialysemaschine nur hergestellt, wenn die richtigen Konnektoren eingelegt wurden. Andernfalls macht die Dialysemaschine den Benutzer darauf aufmerksam, dass die falschen Konnektoren eingelegt wurden.

### 2. Die Dialysemaschine

Die einzelnen Komponenten einer Dialysemaschine sollen nun im folgenden anhand von zwei Ausführungsbeispielen näher beschrieben werden.

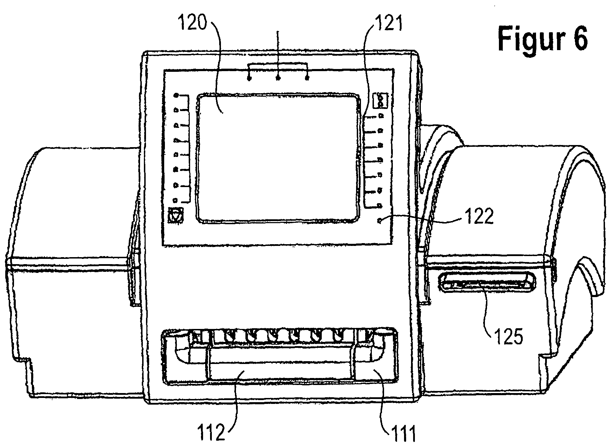

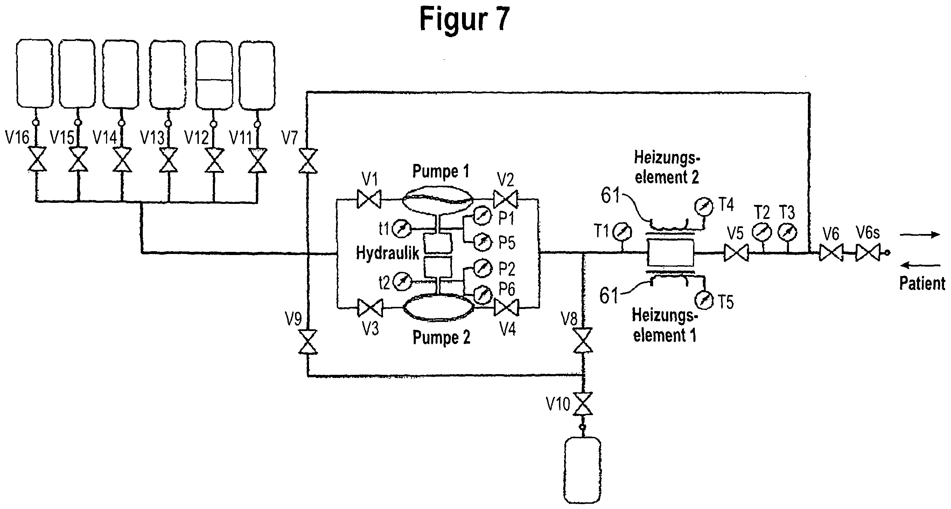

In Figur 6 ist dabei ein erstes Ausführungsbeispiel einer Dialysemaschine gezeigt, bei welchem das erste Ausführungsbeispiel einer Kassette eingesetzt wird. Das sich aus dem ersten Ausführungsbeispiel einer Dialysemaschine und dem ersten Ausführungsbeispiel einer Kassette ergebende Peritonealdialysesystem ist dabei in Figur 7 gezeigt.

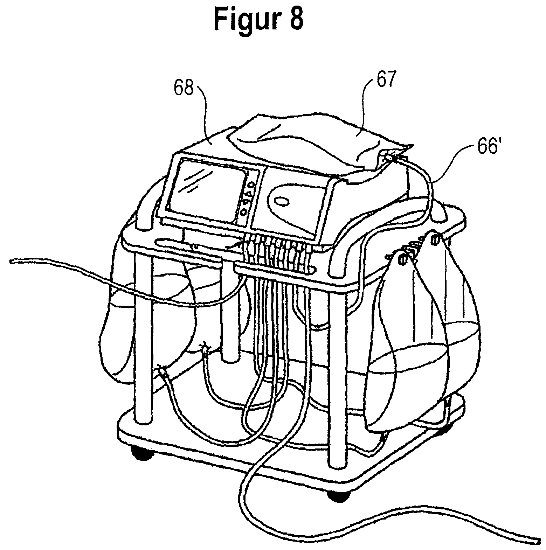

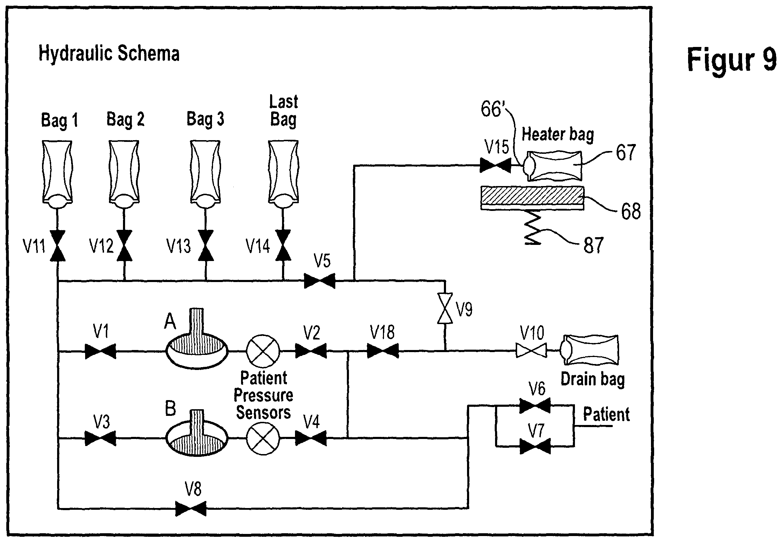

In Figur 8 ist ein zweites Ausführungsbeispiel einer Dialysemaschine gezeigt, bei welcher das zweite Ausführungsbeispiel einer Kassette zum Einsatz kommt. Das sich aus der Kombination des zweiten Ausführungsbeispiels einer Dialysemaschine und des zweiten Ausführungsbeispiels einer Kassette ergebende Dialysesystem ist dann in Figur 9 gezeigt.

Die beiden Ausführungsbeispiele unterscheiden sich dabei zum einen in der Ausgestaltung der Heizung, in der Kopplung zwischen der Dialysemaschine und der Kassette sowie in der Ausgestaltung der Aktoren und Sensoren.

### 2.1 Heizung

Das frische Dialysat muss auf Körpertemperatur gebracht werden, bevor es in den Bauchraum des Patienten befördert wird. Hierfür weist die Dialysemaschine eine entsprechende Heizung auf.

Die Heizung erfolgt dabei üblicherweise elektrisch über ein oder mehrere Heizelemente. Bei den Heizelementen kann es sich dabei z. B. um keramische Heizelemente handeln. Bei solchen keramischen Heizelementen ist eine Widerstandsbahn auf einem Keramikträger aufgebracht. Durch Anlegen einer Spannung an die Widerstandsbahn wird diese erhitzt, wodurch sich auch das keramische Trägermaterial erhitzt. Das keramische Heizelement ist dabei üblicherweise auf einer Heizplatte angeordnet. Diese kann beispielsweise aus Aluminium gefertigt sein. An die Heizplatte werden wiederum die Fluidwege angekoppelt, so dass das in den Fluidwegen befindliche Dialysat erhitzt werden kann.

Zum Erwärmen der Flüssigkeit stehen zwei unterschiedliche Ausgestaltungen zur Verfügung. Zum einen kann zunächst eine größere Menge an Dialysat erwärmt werden, welche erst nach der Aufwärmphase zum Patienten gepumpt wird. Dies erfolgt üblicherweise über einen Heizbeutel, welcher auf einer Heizplatte des Dialysegerätes aufliegt.

Bei dem Heizbeutel kann es sich dabei um den Dialysatbeutel handeln, in welchem das Dialysat zur Verfügung gestellt wird. Üblicherweise wird jedoch ein separater Heizbeutel eingesetzt, in welchem das Dialysat zum Aufheizen hineingepumpt wird. Ist das Dialysat im Heizbeutel erwärmt, wird es von dort aus zum Patienten gepumpt.

Ein solches Konzept ist bei den in Figuren 8 und 9 gezeigten zweiten Ausführungsbeispiel einer Dialysemaschine verwirklicht. Dabei ist ein Heizbeutel 67 vorgesehen, welcher auf einer Heizplatte 68 aufliegt. Die Heizplatte 68 ist dabei auf der Oberseite des Peritonealdialysegerätes angeordnet, so dass sie leicht zugänglich ist. Der Heizbeutel 67 ist dabei über eine Leitung 66' mit der Kassette verbunden. Die Kassette weist dabei die Ventile V5, V9 und V15 auf, über welche der Heizbeutel 67 mit den übrigen Komponenten des Fluidsystems verbunden werden kann. So kann frisches Dialysat von den Dialysatbehältern 10 über die Pumpkammern zum Heizbeutel 67 gepumpt werden. Zu Beginn einer Behandlung wird also zunächst der Heizbeutel 67 mit kaltem Dialysat gefüllt. Das Dialysat im Heizbeutel 67 wird dann über die Heizplatte 68 auf Körpertemperatur erwärmt. Daraufhin wird das Dialysat über die Pumpkammern zum Patienten gepumpt. Daraufhin kann der Heizbeutel 67 wieder befüllt werden, so dass die für den nächsten Behandlungszyklus benötigte Dialysatmenge erhitzt werden kann.

Vorteilhafterweise ist dabei im Bereich der Heizplatte 68 ein Temperatursensor 88 vorgesehen, welcher mit dem Heizbeutel 67 in Kontakt steht und so die Temperatur des Dialysats im Heizbeutel 67 messen kann. Weiterhin kann ein Temperatursensor an der Heizplatte oder am Heizelement vorgesehen sein, welcher die Temperatur des Heizelements oder der Heizplatte misst. Eine entsprechende Steuerung sorgt nun dafür, dass die Heizplatte nicht zu heiß für das Material des Beutels wird.

Der Heizbeutel 67 kann zudem Funktionen bei der Billanzierung der Flüssigkeitsströme übernehmen. So kann die Heizplatte 68 Teil einer Waage 87 sein, über welche das Gewicht des Heizbeutels 67 bestimmbar ist. Hierdurch kann die Flüssigkeitsmenge, welche nach dem Erwärmen dem Patienten zugeführt wird, bestimmt werden.

Alternativ zu der beim zweiten Ausführungsbeispiel gezeigten Erwärmung des Dialysats über einen Heizbeutel kann das Dialysat auch erwärmt werden, während es zum Patienten gepumpt wird. Die Heizung arbeitet damit in Form eines Durchlauferhitzers, welcher das durch das Fluidsystem bewegte Dialysat erwärmt, während es durch die Fluidwege gepumpt wird.

Bei diesem Konzept ist ein Dialysatkanal vorgesehen, welcher an ein Heizelement der Dialysemaschine gekoppelt wird. Während das Dialysat durch den Dialysatkanal fließt, nimmt es dabei Wärme von dem Heizelement der Dialysemaschine auf.

Ein solches Konzept ist bei dem ersten Ausführungsbeispiel einer Dialysemaschine, welches in Figuren 6 und 7 gezeigt ist, implementiert. Dabei ist der Heizbereich in die Kassette integriert, wie dies bereits oben dargestellt wurde. Beim Ankoppeln der Kassette an die Dialysemaschine kommt dabei der Heizbereich der Kassette mit Heizelementen der Dialysemaschine thermisch in Kontakt.

Die Heizelemente können dabei ebenfalls als keramische Heizelemente ausgeführt sein und mit Heizplatten in Kontakt stehen, welche dann an den Heizbereich der Kassette gekoppelt werden. Wie bereits hinsichtlich der Kassette dargestellt, steht dabei sowohl mit der Oberseite als auch mit der Unterseite des Heizbereichs jeweils eine Heizplatte in Kontakt, welche das durch den Heizbereich fließende Dialysat erwärmt.

Am Zulauf und am Ablauf des Heizbereichs sind jeweils Temperatursensorbereiche in der Kassette vorgesehen, welche durch das Ankoppeln der Kassette mit Temperatursensoren der Peritonealdialyse in Kontakt kommen. Durch die Temperatursensoren T1 bis T3 kann so die Temperatur des in den Heizbereich einfließenden Dialysats sowie die Temperatur des aus dem Heizbereich herausfließenden Dialysats bestimmt werden. Weiterhin sind Temperatursensoren T4 und T5 vorgesehen, welche die Temperatur der Heizelemente und/oder der Heizplatten bestimmen.

Die Verwendung von mindestens zwei Heizelementen ermöglicht es dabei, die Heizelemente jeweils so zusammenzuschalten, dass sie bei einer Versorgungsspannung von 220 V im wesentlichen die gleiche Leistung abgeben wird wie bei einer Versorgungsspannung von 110 V. Hierfür werden die beiden Heizelemente bei 110 V in einer Parallelschaltung betrieben, während sie bei einer Versorgungsspannung von 220 V in einer Seriellschaltung betrieben werden. Eine solche Anpassung der Verschaltung der Heizelemente an die Versorgungsspannung kann dabei unabhängig davon, ob die Heizung gemäß dem ersten oder dem zweiten Ausführungsbeispiel erfolgt, implementiert werden.

### 2.2 Ankopplung der Kassette

Um eine Ankopplung der Aktoren und/oder Sensoren der Dialysemaschine an die entsprechenden Bereiche der Kassette zu ermöglichen, weist die Dialysemaschine eine Kassettenaufnahme mit einer Ankoppelfläche auf, an welcher die Kassette angekoppelt werden kann. An der Ankoppelfläche sind die entsprechenden Aktoren, Sensoren und/oder Heizelemente der Dialysemaschine angeordnet. Die Kassette wird mit dieser Ankoppelfläche so verpresst, dass die entsprechenden Aktoren, Sensoren und/oder Heizelemente mit den entsprechenden Bereichen an der Kassette in Kontakt kommen.

Dabei ist vorteilhafterweise an der Ankoppelfläche der Dialysemaschine eine Matte aus einem flexiblen Material vorgesehen, insbesondere eine Silikonmatte. Diese sorgt dafür, dass die flexible Folie der Kassette mit den Stegbereichen der Kassette verpresst wird und so die Fluidwege innerhalb der Kassette voneinander trennt.

Vorteilhafterweise ist weiterhin ein umlaufender Rand der Ankoppelfläche vorgesehen, welcher mit dem Randbereich der Kassette verpresst wird. Vorteilhafterweise erfolgt die Verpressung dabei luftdicht, so dass zwischen der Ankoppelfläche und der Kassette ein Unterdruck aufgebaut werden kann.

Gegebenenfalls kann auch ein Vakuumsystem vorgesehen sein, welches Luft aus dem Raum zwischen Ankoppelfläche und Kassette abpumpen kann. Hierdurch wird eine besonders gute Ankopplung der Aktoren, Sensoren und/oder Heizelemente des Peritonealdialysegerätes mit den entsprechenden Bereichen der Kassette ermöglicht. Zudem erlaubt das Vakuumsystem eine Dichtigkeitsprüfung der Kassette. Hierfür wird nach dem Ankoppeln ein entsprechendes Vakuum angelegt und überprüft, ob dieses Vakuum aufrechterhalten wird.

Das Anpressen der Kassette erfolgt z. B. pneumatisch. Hierzu ist üblicherweise ein Luftkissen vorgesehen, welches mit Druckluft befüllt wird und so die Kassette an die Ankoppelfläche anpresst.

Die Kassettenaufnahme weist üblicherweise eine der Ankoppelfläche gegenüberliegende Aufnahmefläche auf, in welche das Hartteil der Kassette eingelegt wird. Die Aufnahmefläche weist hierfür vorteilhafterweise entsprechende Vertiefungen auf. Die Aufnahmefläche mit der eingelegten Kassette kann dann über eine pneumatische Anpressvorrichtung an die Ankoppelfläche angepresst werden.

Das Einlegen der Kassette kann dabei in unterschiedlicher Weise geschehen. Im ersten Ausführungsbeispiel einer Dialysemaschine, welches in Figur 6 gezeigt ist, ist hierfür eine Schublade 111 vorgesehen, welche aus der Dialysemaschine ausgefahren werden kann. In diese Schublade wird die Kassette eingelegt. Die Kassette wird dann zusammen mit der Schublade in die Dialysemaschine eingeschoben. Daraufhin erfolgt das Verpressen der Kassette mit der Ankoppelfläche, welche im Inneren des Gerätes angeordnet ist. Dabei werden Kassette und Koppelfläche zunächst mechanisch aneinander gefahren und dann pneumatisch miteinander verpresst.

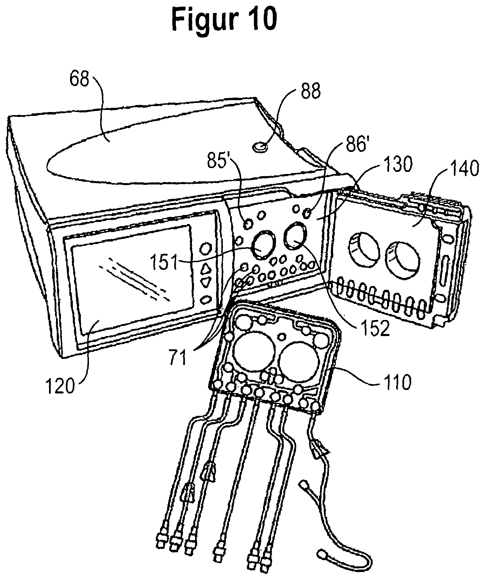

Das Ankoppeln einer Kassette 110 gemäß dem zweiten Ausführungsbeispiel ist in Figur 10 näher dargestellt. Die Ankoppelfläche 130 ist durch Öffnen einer Tür 140 frei zugänglich, so dass die Kassette in der richtigen Position an der Ankoppelfläche 130 angeordnet werden kann. Die Ankoppelfläche 130 ist dabei nach hinten zur Vertikalen geneigt, was ein leichteres Ankoppeln ermöglicht. Nun kann die Tür 140 geschlossen werden, so dass eine Aufnahmefläche an der Tür mit der Rückseite der Kassette in Kontakt kommt. Das Verpressen erfolgt nun durch ein an der Tür angeordnetes Luftkissen. Zudem wird ein Vakuum zwischen die Ankoppelfläche und die Kassette 110 angelegt.

Das erste Ausführungsbeispiel einer Dialysemaschine weist weiterhin eine Vorrichtung zum automatischen Konnektieren auf. Hierfür ist eine Konnektorenaufnahme 112 vorgesehen, in welche die Konnektoren der Dialysatbeutel 10 eingelegt werden. Die Konnektorenaufnahme 112 fährt dann in das Gerät hinein, wo ein Barcodeleser vorgesehen ist, welcher die auf den Konnektoren aufgebrachten Barcodes liest. So kann das Gerät überprüfen, ob die richtigen Beutel eingelegt wurden. Werden die richtigen Beutel erkannt, so fährt die Konnektoraufnahme 112 komplett ein und schließt so die Konnektoren der Beutel an den als Konnektoren ausgeführten Anschlüssen 11 der Kassette an.

Im zweiten Ausführungsbeispiel wurde auf eine solche automatische Konnektierung dagegen verzichtet. Daher sind an den Anschlüssen 11 der Kassette Schlauchabschnitte angeordnet, welche manuell über Konnektoren mit den entsprechenden Beuteln verbunden werden müssen.

### 2.3 Pumpaktoren

Das Pumpen der Flüssigkeit durch das Fluidsystem erfolgt in den Ausführungsbeispielen durch eine Membranpumpe, welche von den Pumpkammern 53 und 53' zusammen mit der flexiblen Folie der Kassette gebildet wird. Wird die flexible Folie dabei durch einen entsprechenden Pumpaktor in die Pumpkammer hineingedrückt, so wird Flüssigkeit aus der Pumpkammer in die geöffneten Bereiche der Fluidwege der Kassette gepumpt. Umgekehrt wird durch Herausziehen der Folie aus der Pumpkammer Fluid aus den Fluidwegen in die Pumpkammer gesaugt.

Der Pumphub erfolgt dabei durch Bewegen eines Pumpaktors in die Pumpkammer. Für den Saughub wird der Pumpaktor wieder von der Pumpkammer wegbewegt. Durch die luftdichte Verpressung von Kassette und Ankoppelfläche entsteht dabei ein Unterdruck, durch welchen die flexible Folie der Kassette dem Pumpaktor folgt und so wieder aus der Pumpkammer herausgezogen wird.

Um eine gute Ankopplung des Pumpaktors an die flexible Folie der Kassette zu ermöglichen, kann zudem ein Vakuumsystem vorgesehen sein. Über das Einstellen eines entsprechenden Vakuums zwischen der Ankoppelfläche und der Kassette kann dabei insbesondere die Kraft eingestellt werden, mit welcher die flexible Folie während eines Saughubs maximal von der Pumpkammer wegbewegt wird.

Hierdurch kann die Saugkraft der Pumpe sehr fein eingestellt werden. Die Pumpkraft wird dagegen durch die Schubkraft des Aktors eingestellt.

Die Bilanzierung der Flüssigkeitsströme kann dabei durch das Zählen der Saug- und Pumphübe erfolgen, da die Membranpumpe eine hohe Genauigkeit der mit jedem Hub gepumpten Flüssigkeitsmenge aufweist.

### 2.3.1. Hydraulischer Antrieb

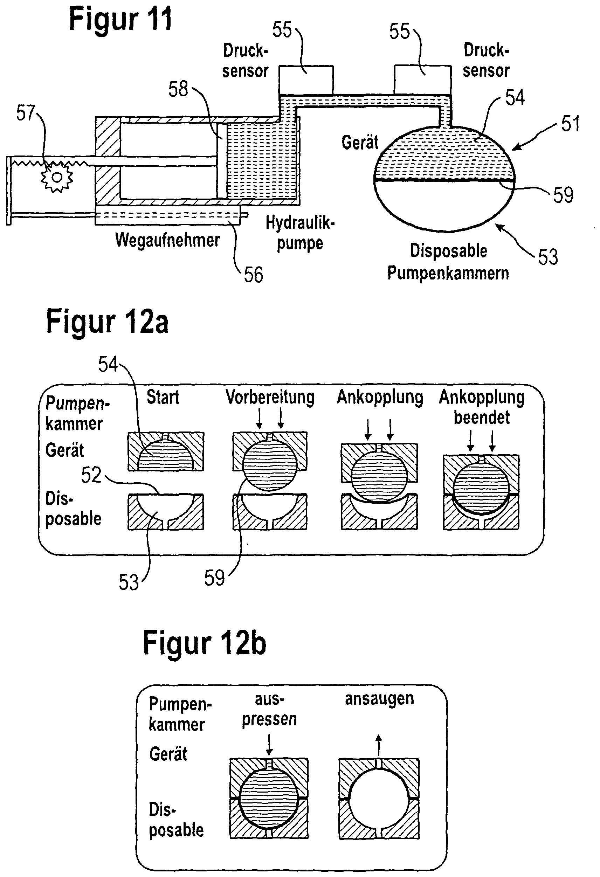

Der Aufbau eines ersten Ausführungsbeispiels eines Pumpaktors ist in Figur 11 gezeigt. Der Pumpaktor wird dabei hydraulisch bewegt. Hierfür ist eine Membran 59 vorgesehen, welche an der flexiblen Folie der Kassette anlegt. Die Membran 59 kann dabei z. B. aus Silikon gefertigt sein. Hinter der Membran 59 ist eine Kammer 54 vorgesehen, welche mit Hydraulikfluid gefüllt werden kann. Durch Anlegen eines Überdrucks in der Kammer 54 wird die Membran 59 und mit dieser die flexible Folie in die Pumpkammer 53 der Kassette hineingedrückt. Durch Anlegen eines Unterdruckes an die Kammer 54 wird die Membran 59 dagegen in die Kammer 54 hineingezogen. Durch den Unterdruck zwischen der flexiblen Folie und der Membran folgt die flexible Folie dieser Bewegung, so dass sich das Volumen der Pumpkammer 53 vergrößert. Der Pumpvorgang mit den Pumphub und dem Ansaughub ist dabei schematisch in Figur 12b dargestellt.

Zum Betrieb der Pumphydraulik ist eine Hydraulikpumpe 58 vorgesehen. Diese weist einen Zylinder auf, in welchem ein Kolben über einen Motor 57 hin und her bewegt werden kann. Hierdurch wird die Hydraulikflüssigkeit über eine entsprechende Verbindungsleitung in die Kammer 54 hineingepreßt oder aus dieser wieder herausgesogen. An der Hydraulikpumpe 58 ist dabei ein Wegaufnehmer 56 vorgesehen, über welchen die Bewegung des Kolbens aufgenommen werden kann. Hierdurch kann bestimmt werden, wieviel Hydraulikflüssigkeit in die Kammer 54 hineingepreßt bzw. wieviel Hydraulikflüssigkeit aus dieser entnommen wurde. Weiterhin sind Drucksensoren 55 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 56 verglichen werden können und hierdurch die Dichtigkeit des Hydrauliksystems überprüft werden kann.

Zudem ermöglichen die Drucksensoren eine Bestimmung des Drucks in der Pumpekammer 53 der Kassette. Wird die Hydraulikpumpe 58 nicht bewegt, so stellt sich ein Druckgleichgewicht zwischen der Kammer 54 und der Pumpkammer 53 ein. Der Druck der Hydraulikflüssigkeit entspricht damit dem Druck in der Pumpenkammer 53.

In Figur 12a ist nun der Ankoppelvorgang des Pumpenaktors an die Pumpenkammer 53 gezeigt. Zur Vorbereitung des Ankoppelns wird dabei zunächst die Kammer 54 mit Hydraulikfluid beaufschlagt, dass sich die Membran 59 nach außen wölbt. Daraufhin werden Ankoppelfläche und Kassette aufeinander zu bewegt, so dass die Membran 59 die flexible Folie der Kassette in die Pumpkammer 53 hineindrückt. Nach dem Verpressen von Ankoppelfläche und der Kassette ist der Raum zwischen der Membran und der flexiblen Folie nach außen luftdicht abgeschlossen, so dass die flexible Folie der Bewegung der Membran folgt. Dies ist in Figur 12b gezeigt.

Der in Figur 11 gezeigte Pumpaktor ist dabei beim ersten Ausführungsbeispiel einer Dialysemaschine implementiert, wie dies auch aus Figur 7 ersichtlich ist. Dabei ist für jede der beiden Pumpkammern 53 und 53' jeweils ein entsprechender Pumpaktor vorgesehen.

### 2.3.2 Elektromechanischer Antrieb

Alternativ kann der Pumpaktor auch elektromotorisch betrieben werden. Hierfür ist ein entsprechend geformter Stempel vorgesehen, welcher über einen Elektromotor, insbesondere über einen Schrittmotor gegen die flexible Folie gedrückt bzw. von dieser wegbewegt wird und so den Pump- bzw. Saughub erzeugt. Solche Pumpaktoren 151 und 152 sind bei dem Ausführungsbeispiel in Fig. 10 gezeigt. Vorteilhafterweise ist dabei ein Vakuumsystem vorgesehen, welches dafür sorgt, dass die flexible Folie dem Stempel auch bei der Saugbewegung folgt.

### 2.4 Ventilaktoren

Als Ventilaktor kann ein Ventilstößel vorgesehen sein, welcher die flexible Folie der Kassette in eine entsprechende Kammer des Hartteils hineindrückt und so den Fluidkanal in diesem Bereich schließt. Der Ventilaktor kann dabei Z.B. pneumatisch betätigt werden. Der Stößel kann dabei über eine Feder vorgespannt sein, so dass er entweder drucklos öffnet oder drucklos schließt.

Alternativ kann der Ventilaktor über eine flexible Membran implementiert werden, welche hydraulisch oder pneumatisch bewegt wird. Die flexible Membran wird dabei durch Anlegen von Druck gegen die Kassette bewegt und drückt so einen entsprechenden Ventilbereich der flexiblen Folie in einen Fluidkanal, um diesen zu verschließen.

Ventilaktoren 71, welche an die Ventilbereiche V1 bis V16 der Kassette angekoppelt werden, sind in Fig. 10 auf der Ankoppelfläche erkennbar.

### 2.5 Sensoren

Die Dialysemaschine weist Sensoren auf, über welche die Maschine angesteuert bzw. deren ordnungsgemäßes Funktionieren überwacht werden kann.

Zum einen sind dabei ein oder mehrere Temperatursensoren vorgesehen, über welche die Temperatur des Dialysats und/oder der Heizelemente gemessen werden kann. Im ersten Ausführungsbeispiel sind die Temperatursensoren dabei an der Ankoppelfläche zur Kassette angeordnet und können so die Temperatur des durch die Kassette fließenden Dialysats messen. Im zweiten Ausführungsbeispiel ist dagegen ein Temperatursensor 88 auf der Heizplatte 68 vorgesehen, welcher die Temperatur des im Beutel 67 befindlichen Dialysats mißt. Weiterhin können an dem oder den Heizelementen Temperatursensoren vorgesehen sein.

Weiterhin können ein oder mehrere Drucksensoren vorgesehen sein, um den Druck in den Pumpkammern zu bestimmen. Hierdurch kann verhindert werden, das Dialysat mit zu hohem Druck zum Patienten gepumpt wird oder der Saugdruck beim Absaugen von Dialysat vom Patienten zu hoch wird.

Im ersten Ausführungsbeispiel erfolgt die Druckmessung dabei über Drucksensoren in der Hydraulik der Pumpaktoren, wie dies oben dargestellt wurde. Im zweiten Ausführungsbeispiel sind dagegen Drucksensoren 85' und 86' in der Ankoppelfläche vorgesehen, welche den Druck in entsprechenden Druckmessbereichen der Kassette direkt messen. Die Ankopplung dieser Drucksensoren an die Kassette wird dabei vorteilhafterweise durch ein Vakuumsystem sichergestellt.

### 2.6 Ein-/Ausgabeeinheit

Die Dialysemaschine umfasst weiterhin eine Ein-/Ausgabeeinheit zur Kommunikation mit einem Bediener. Zur Ausgabe von Informationen ist dabei eine entsprechende Anzeige vorgesehen, welche z. B. durch Leuchtdioden, LCD-Anzeigen oder einen Bildschirm implementiert sein kann. Zur Eingabe von Befehlen sind entsprechende Eingabeelemente vorgesehen. Hierbei können z. B. Drucktasten und Schalter vorgesehen sein.

In beiden Ausführungsbeispielen ist dabei ein Touchscreen 120 vorgesehen, welcher eine interaktive Menüführung ermöglicht. Weiterhin sind Anzeigeelemente 121 und 122 vorgesehen, welche Zustände der Dialysemaschine kompakt darstellen.

Das erste Ausführungsbeispiel weist weiterhin einen Kartenleser 125 auf, über welchen eine Patientenkarte eingelesen werden kann. Auf der Patientenkarte können Daten zur Behandlung des jeweiligen Patienten abgespeichert sein. Hierdurch kann der Behandlungsablauf für den jeweiligen Patienten individuell festgelegt werden.

Die Peritonealdialyse weist weiterhin eine akustische Signaleinheit auf, über welche akustische Signale abgegeben werden können. Insbesondere kann dabei ein akustisches Warnsignal ausgegeben werden, wenn ein Fehlzustand registriert wird. Dabei ist vorteilhafterweise ein Lautsprecher vorgesehen, über welchen die akustischen Signale erzeugt werden können.

### 2.7 Steuerung

Die Peritonealdialyse weist weiterhin eine Steuerung auf, durch welche sämtliche Komponenten angesteuert und überwacht werden. Die Steuerung sorgt dabei für den automatischen Ablauf der Behandlung.

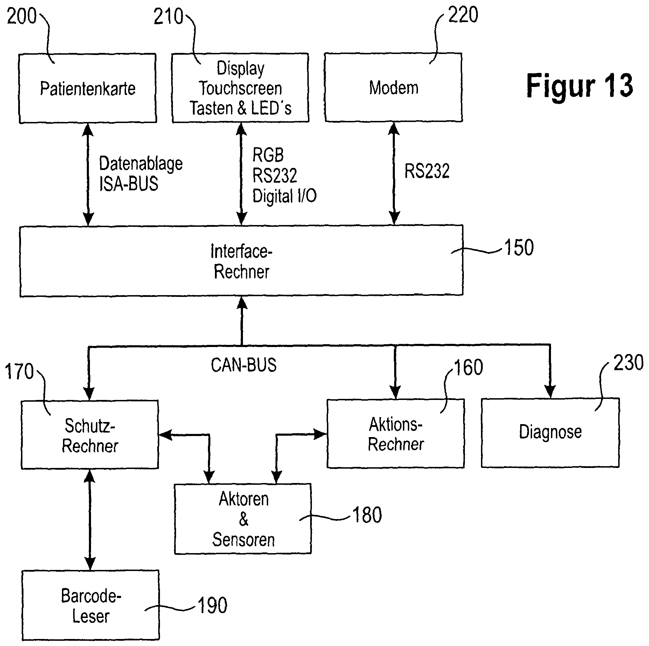

In Figur 13 ist nun der prinzipielle Aufbau eines Ausführungsbeispiels einer solchen Steuerung dargestellt.

Die Kommunikation mit dem Bediener sowie mit externen Informationsquellen erfolgt dabei über einen Interface-Rechner 150. Dieser kommuniziert mit einem Patientenkartenleser 200, einer Ein- und Ausgabeeinheit 210, welche der Kommunikation mit dem Patienten dient, sowie mit einem Modem 220. Über das Modem 220 kann z. B. eine aktualisierte Software aufgespielt werden.

Der Interface-Rechner 150 steht über einen internen Bus mit einem Aktionsrechner 160 und einem Schutzrechner 170 in Verbindung. Der Aktionsrechner 160 und der Schutzrechner 170 erzeugen eine Redundanz des Systems. Der Aktionsrechner 160 erhält dabei Signale von den Sensoren des Systems und berechnet die Steuersignale für die Aktoren 180. Der Schutzrechner 170 erhält ebenfalls Signale von den Sensoren 180 und überprüft, ob die vom Aktionsrechner 160 ausgegebenen Befehle korrekt sind. Stellt der Schutzrechner 170 einen Fehler fest, so leitet er eine entsprechende Notfallprozedur ein. Insbesondere kann der Schutzrechner 170 dabei ein Alarmsignal auslösen. Weiterhin kann der Schutzrechner 170 den Zugang zum Patienten schließen. Hierfür ist ein spezielles Ventil am patientenseitigen Ausgang der Kassette angeordnet, auf welches nur der Schutzrechner 170 Zugriff hat. Dieses Sicherheitsventil ist dabei im drucklosen Zustand geschlossen, so dass es bei einem Ausfall der Pneumatik automatisch schließt.

Der Schutzrechner 170 steht weiterhin mit dem Barcodeleser 190 in Verbindung, und überprüft so den Anschluss der korrekten Dialysatbeutel.

Weiterhin ist ein Diagnosesystem 230 vorgesehen, über welches Fehler des Systems ermittelt und behoben werden können.

### 3. Implementierung der Erfindung

Ein Ausführungsbeispiel der vorliegenden Erfindung, welches bei einem oben dargestellten Dialysesystemen bzw. bei einer der oben dargestellten Dialysemaschinen zum Einsatz kommt, wird nun im folgenden dargestellt. Dabei kann das Ausführungsbeispiel der vorliegenden Erfindung mit einzelnen oder mehreren Komponenten, wie sie oben beschrieben wurden, kombiniert werden.

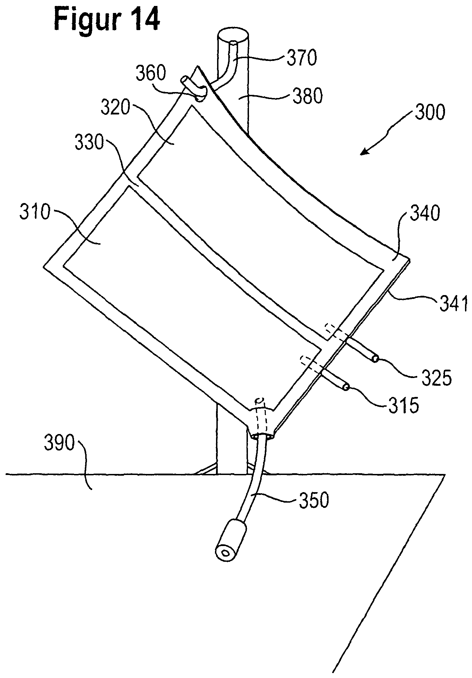

In Figur 14 ist ein Ausführungsbeispiel eines Mehrkammerbehälters gezeigt, wie er bereits in Abschnitt 1.1 hinsichtlich der Ausführung eines Dialysatbehälters beschrieben wurde. Vorteilhafterweise handelt es sich dabei bei dem in Figur 14 gezeigten Ausführungsbeispiel ebenfalls um einen solchen Dialysatbehälter.

Der Mehrkammerbehälter 300 weist dabei im Ausführungsbeispiel eine erste Kammer 310 und eine zweite Kammer 320 auf, welche durch eine Trennanordnung 330 voneinander getrennt sind. In der ersten Kammer 310 ist dabei eine erste Einzellösung, in der zweiten Lösung 320 eine zweite Einzellösung gelagert. Die Trennanordnung 330 kann mechanisch geöffnet werden, so dass die beiden Kammern 310 und 320 fluidisch miteinander in Verbindung stehen und so die beiden Einzellösungen zu einer Gesamtlösung vermischt werden können. Insbesondere kann die Trennanordnung dabei manuell durch den Patienten oder eine Bedienperson geöffnet werden. Insbesondere bilden die beiden Kammern 310 und 320 nach dem Öffnen der Trennanordnung 330 eine gemeinsame entsprechend größere Kammer. Jede der beiden Kammern 310 und 320 weist einen Befüllstutzen 315 bzw. 325 auf, über welchen die jeweilige Kammer mit einer Einzellösung befüllt werden kann. Weiterhin weist der Mehrkammerbehälter 300 einen Ablauf 350 auf, aus welchem die nach Öffnen der Trennanordnung 330 entstehende Gesamtlösung entnommen werden kann. Der Ablauf 350 ist dabei im Bereich der ersten Kammer 310 angeordnet. Wird die Trennanordnung 330 daher nicht geöffnet, so fließt aus dem Ablauf 350 lediglich Flüssigkeit aus der ersten Kammer 310, d. h. die erste Einzellösung.

Die Trennanordnung 330 kann im Ausführungsbeispiel durch die Ausübung von Druck auf mindestens eine der beiden mit Flüssigkeit gefüllten Kammern 310 oder 320 geöffnet werden. Im Ausführungsbeispiel wird der Mehrkammerbehälter dabei von einem Mehrkammerbeutel gebildet. Dieser besteht aus zwei Folienschichten 340 und 341, welche in einem Randbereich miteinander verbunden, insbesondere miteinander verschweißt sind. Die Trennanordnung 330 wird dabei von einer sogenannten Peel-Naht gebildet, welche von einem ersten Randbereich zu einem zweiten Randbereich des Beutels reicht und so den Beutel in zwei Kammern aufteilt. Im Bereich der Peel-Naht sind die beiden Folienschichten 340 und 341 ebenfalls miteinander verbunden, so dass der Mehrkammerbeutel eine getrennte Lagerung der Einzellösungen erlaubt. Die Verbindung im Bereich der Peel-Naht 330 ist jedoch so ausgeführt, dass sie durch Druck auf mindestens eine der Kammern 310 oder 320 geöffnet werden kann. Hierdurch verbinden sich die beiden Kammern 310 und 320 zu einer gemeinsamen größeren Kammer.

Der Ablauf 350 wird von einem Schlauchabschnitt gebildet, welcher zwischen die beiden Folienlagen 340 und 341 eingeschweißt ist und im Bereich der ersten Kammer 310 angeordnet. Der Ablauf 350 ist dabei üblicherweise mit einem Konnektor ausgestattet, über welchen weiteren Komponenten des Fluidsystems angeschlossen werden kann, insbesondere an eine Kassette.

Gegebenenfalls kann eine weitere Peel-Naht vorgesehen sein, welche den Ablauf 350 zunächst von der ersten Kammer oder von den beiden Kammern mit Einzellösungen abtrennt. Eine solche Peel-Naht muss daher geöffnet werden, um überhaupt Flüssigkeit aus dem Beutel entnehmen zu können und stellt damit ein weiteres Sicherheitselement dar.

Der Mehrkammerbeutel 300 weist auf der gegenüberliegenden Seite des Ablaufs 350 ein Befestigungselement z.B. in Form einer Aussparung 360 auf, an welcher der Mehrkammerbeutel z. B. an einem Haken 370 aufgehängt werden kann. Dies stellt sicher, dass der Ablauf 350 an der niedrigsten Position des Beutels angeordnet ist und so die Flüssigkeit aus dem Beutel komplett ablaufen kann. Hierfür ist der Ablauf 350 in einer Ecke des im wesentlichen rechteckigen Beutels, die Aussparung bzw. Öse 360 in der gegenüberliegenden Ecke angeordnet. Die Peel-Naht 330 verläuft dabei so, dass im aufgehängten Zustand des Beutels die zweite Kammer 320 zumindest teilweise höher angeordnet ist als die erste Kammer 310. Hierfür verläuft die Peel-Naht 330 von einem Randbereich des Beutels zu einem zweiten Randbereich des Beutels, wobei sich der erste und der zweite Randbereich jeweils zwischen dem Bereich des Ablaufs 350 und dem Bereich der Aussparung bzw. Öse 360 befinden. Im Ausführungsbeispiel verläuft die Peel-Naht 330 dabei im wesentlichen parallel zu einer Seite des rechteckigen Beutels.

Ein Ausführungsbeispiel, bei welcher die Dialysemaschine automatisch die sachgerechte Öffnung der Trennanordnung 330 anhand eines Drucks im Fluidsystem überprüft, soll nun ebenfalls anhand der Figur 14 näher beschrieben werden. Die Überprüfung der ordnungsgemäßen Öffnung erfolgt dabei durch die Bestimmung des hydrostatischen Drucks der Flüssigkeit aus dem Mehrkammerbehälter 300. Hierfür wird das Fluidsystem an die Dialysemaschine angekoppelt, wodurch ein Drucksensor an einen Druckmessbereich des Fluidsystems angekoppelt wird.

Die Messung des Drucks im Fluidsystem kann dabei z. B. anhand des Drucks einer Hydraulikflüssigkeit, durch welche ein Aktor der Dialysemaschine bewegt wird, oder durch eine direkte Messung des Drucks in einer Kammer der Kassette erfolgen. Die entsprechenden Druckmesssysteme wurden bereist oben ausführlich beschrieben.

Durch Öffnen der entsprechenden Ventilbereiche im Fluidsystem durch eine entsprechende Ansteuerung der Ventilaktoren der Dialysemaschine wird nun eine fluidische Verbindung zwischen dem Druckmessbereich im Fluidsystem und dem Ablauf 350 des Mehrkammerbehälters 300 hergestellt. Die Druckmessung kann dabei nach Befüllen zumindest eines Teils des Fluidsystems erfolgen.

Nun wird im stationären Zustand des Systems der Druck, welcher durch die Flüssigkeitssäule aus dem Mehrkammerbehälter auf den Druckmessbereich ausgeübt wird, bestimmt. Da der Mehrkammerbehälter 300 so angeordnet ist, dass der Flüssigkeitsspiegel in der ersten Kammer 310 bei nicht geöffneter Trennanordnung 330 niedriger liegt als der Flüssigkeitsspiegel bei geöffneter Trennanordnung in der sich bildenden gemeinsamen Kammer, ergibt sich bei geschlossner Trennanordnung ein anderer Druck auf der Druckmessanordnung als nach Öffnung der Trennanordnung. Bei der gezeigten Ausführung wird dabei bei geöffneter Trennanordnung ein höherer hydrostatischer Druck auf den Druckmessbereich lasten.

Um eine zuverlässige Unterscheidung zwischen einem geöffneten und einem nicht geöffneten Zustand der Trennanordnung zu gewährleisten, sollte der Mehrkammerbehälter 300 dabei in einer fest definierten Höhe mit Bezug auf den Druckmessbereich des Fluidsystems angeordnet werden. Hierfür kann der Mehrkammerbehälter 300 z. B. wie in Figur 14 gezeigt an einem Haken 370 einer Halterung 380 angehängt werden, welche ihrerseits mit einem Gestell 390 in Verbindung steht. Auf diesem Gestell ist vorteilhafterweise die Dialysemaschine angeordnet, so dass sich eine genau definierte Position von Mehrkammerbehälter 300 und Dialysemaschine ergibt.

Bei der in Figur 14 gezeigten Anordnung ist dabei der Mehrkammerbehälter 300 neben bzw. oberhalb der Dialysemaschine angeordnet. Die vorliegende Erfindung kann jedoch auch bei einer Anordnung, wie sie in Figur 8 dargestellt ist, eingesetzt werden. Dabei befindet sich der Flüssigkeitspegel in den Dialysatbehältern unterhalb des Druckmessbereiches. Zur Überprüfung der Öffnung der Trennanordnung wird daher zunächst Flüssigkeit aus dem Mehrkammerbehälter 300 in die Fluidwege hineingepumpt und dann der hydrostatische Druck der Flüssigkeit aus dem Mehrkammerbehälter 300 gemessen, welcher in diesem Fall negativ ist.

In einem weiteren Ausführungsbeispiel kann die Trennanordnung von der Dialysemaschine selbsttätig geöffnet werden, und zwar durch Druckbeaufschlagung einer Kammer des Mehrkammerbehälters durch die Dialysemaschine (automatische Öffnung). Die automatische Öffnung kann dabei zum einen über die Pneumatik der Dialysemaschine erfolgen, zum anderen aber auch durch die Pumpaktoren.

Bei der internen automatischen Druckbeaufschlagung der Peel-Naht durch die Pneumatik ist zum Öffnen der Peel-Naht Außenluft notwendig, die über einen Filter (Sterilität) angesaugt und in den Beutel gepumpt werden muss. Dieses Verfahren ist insbesondere dann von Vorteil, wenn nur ein Mehrkammerbeutel benutzt wird. Um die Peel-Naht zu öffnen, ist ein gewisser Überdruck notwendig, welcher bei üblichen Beuteln im Bereich von 50 mbar (relativ) liegt. Das System erzeugt in der Kammer einen solchen Überdruck und öffnet so die Peel-Naht.

Die Überprüfung der Öffnung kann wie folgt erfolgen:
a) ein Druckanstieg über den zum Öffnen notwendigen Wert hinaus, ohne das ein Druckabfall vorher stattgefunden hat, kann als bereits geöffneter Beutel vom System gewertet werden. Hierbei wird ein zweiter Grenzwert vorgegeben, welcher selbstverständlich unterhalb der max. zulässigen Druckbeaufschlagung des Beutels liegen muss.
b) die Charakteristik des Druckanstiegs kann ausgewertet werden, d.h. bei einer geöffneten Peel-Naht im Beutel ist der Druckanstieg langsamer als bei einer geschlossnen Peel-Naht.

Bei der internen automatischen Druckbeaufschlagung der Peel-Naht durch die Pumpaktoren kann bei der Benutzung von zwei oder mehreren Beuteln (Regelfall) die Flüssigkeit von einem Beutel in den anderen gepumpt werden. Gegebenenfalls kann Flüssigkeit auch von einer Kammer des Mehrkammerbeutels in eine andere Kammer erfolgen, wofür die Mehrkammerbeutel jedoch entsprechende Zugänge benötigen. Durch das Pumpen von Flüssigkeit in die Kammer wir der Druck in dieser größer, die Peel-Naht öffnet sich. Eine Kontaminierung durch Außenluft ist hier nicht gegeben. Hierbei kann das gepumpte Volumen und/oder der Druck zur Steuerung herangezogen werden.

Auch die Überwachung der Öffnung kann über die Drucksensorik erfolgen. Der beim Öffnen der Trennanordnung entstehende Druckabfall (üblicherweise im Bereich 50 mbar) innerhalb des Beutels kann leicht detektiert werden, insbesondere über die den Pumpkammern zugeordnete Drucksensorik.

Folgende Parameter können in die Überprüfung der Öffnung einfließen:
a) die Charakteristik des Druckanstiegs kann ausgewertet werden, d.h. bei einer geöffneten Peel-Naht im Beutel ist der Druckanstieg langsamer als bei einer geschlossnen Peel-Naht.
b) zusätzliche Bewertung des Füllvolumens zur Öffnung der Peel-Naht, d.h. nach Volumen X muss die sich Peel-Naht im Beutel öffnen und der Druck abfallen.

Die automatische Öffnung der Trennanordnung kann weiterhin auch mit anderen Verfahren zur Überprüfung der Öffnung der Trennanordnung kombiniert werden.

Die automatische Öffnung der Trennanordnung kann dabei in der Füllen-Prozedur des Systems mit abgehandelt werden. Ein Fördern der Flüssigkeit in die anderen Lösungsbeutel kann hier einfach implementiert werden. Ungemischte Flüssigkeiten, die nach dem Öffnen der Trennanordnung im Set verbleiben, könnten in die Drainage gefördert werden.

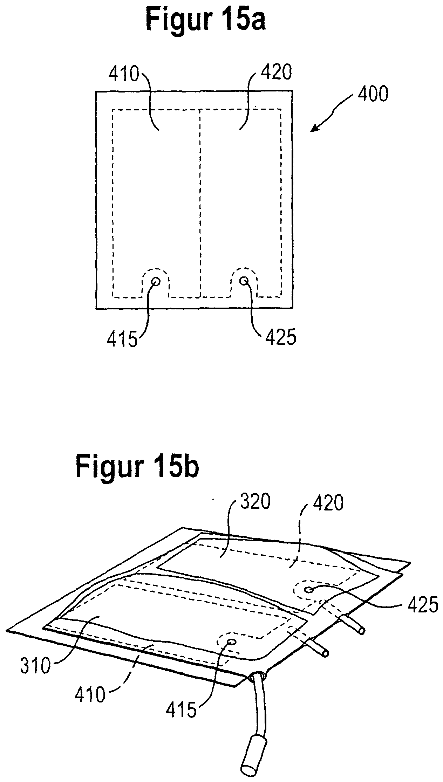

Zwei Ausführungsbeispiele der vorliegenden Erfindung sollen nun anhand der Figuren 15a und 15b näher dargestellt werden. Erfindungsgemäß erfolgt die Überprüfung der sachgerechten Öffnung der Trennanordnung über die Bestimmung zumindest eines Temperaturwertes des Fluidsystems.

In 15a ist dabei eine Heizanordnung 400 gemäß einem Ausführungsbeispiel der vorliegenden Erfindung gezeigt. Die Heizanordnung 400 weist dabei eine Heizfläche auf, an welche der Mehrkammerbehälter angekoppelt werden kann. Das Ankoppeln erfolgt dabei im einfachsten Fall durch Auflegen des Mehrkammerbehälters auf die Heizfläche. Die Heizfläche kann dabei so angeordnet sein, wie dies in Figur 8 anhand des dort gezeigten zweiten Ausführungsbeispiels einer Dialysemaschine bereits dargestellt wurde. Insbesondere kann die Heizfläche dabei in eine Wagschale der Wägeeinrichtung der Dialysemaschine integriert werden. Anders als dort wird jedoch nicht ein separater Heizbeutel, sondern der Mehrkammerbeutel selbst auf die Heizfläche aufgelegt und beheizt. Die Heizfläche weist nun einen ersten Heizbereich 410 und einen zweiten Heizbereich 420 auf. Dabei kann zumindest der erste Heizbereich 410 getrennt von dem zweiten Heizbereich 420 erwärmt werden. Die Heizbereiche 410 und 420 sind dabei so ausgeführt, dass der erste Heizbereich 410 an die erste Kammer 310 des Mehrkammerbehälters und der zweite Heizbereich 420 an die zweite Kammer 320 des Mehrkammerbehälters ankoppelt. Wie in Figur 15b gezeigt, kann dies realisiert werden, indem die erste Kammer 310 auf dem ersten Heizbereich 410 aufliegt, während die zweite Kammer 320 auf dem zweiten Heizbereich 420 aufliegt.

Wie oben dargestellt, kann die Heizfläche im wesentlichen waagrecht angeordnet werden, so dass der Mehrkammerbeutel auf der Heizfläche aufliegt und von dieser gehalten wird. Alternativ kann die Heizfläche mit den Sensoren jedoch auch im Bereich einer Beutelaufhängung angeordnet sein, wobei der Mehrkammerbeutel an der Heizfläche anliegt. Dabei kann eine in eine Anlagefläche integrierte Heizfläche mit Temperatursensoren vorgesehen sein, welche unterhalb eines Hakens zum Aufhängen des Beutels angeordnet ist und an welcher der Beutel durch die Schwerkraft anliegt. Die Anlagefläche kann dabei senkrecht oder leicht schräg angeordnet sein, wobei der Beutel durch seine Wölbung an der Anlagefläche anliegt. Die Zuordnung der Heizelemente und Sensoren zu den Kammern des Beutels erfolgt dabei in der gleichen Weise, wie dies oben bezüglich der im wesentlichen waagrecht angeordneten Heizfläche dargestellt wurde. Die beiden Heizbereiche verlaufen dabei vorteilhafterweise schräg, so dass der Merkammerbeutel an einer Ecke aufgehängt werden kann und die beiden hierdurch schräg angeordneten Kammern den beiden Heizbereichen zugeordnet sind.

Bei den oben beschriebenen Ausführungsformen sind die Temperatursensoren in die Heizfläche integriert und müssen mit dem Mehrkammerbeutel in Kontakt gebracht werden. Es ist jedoch auch denkbar, eine oder mehrere Temperatursensoren direkt am Mehrkammerbeutel anzuordnen. Der oder die Temperatursensoren können so sicher den jeweiligen Kammern zugeordnet werden. Dabei können der oder die Temperatursensoren an einer Stelle plaziert werden, welche nicht mit den Heizelementen in Kontakt kommt, z.B. auch der der Heizfläche gegenüberliegenden Wand des Beutels.

Insbesondere können solche Sensoren auf den Mehrkammerbeutel aufgeklebt oder anderweitig in den Mehrkammerbeutel integriert werden. Dabei ist denkbar, dass die Temperatursensoren drahtlos mit der Dialysemaschine kommunizieren, insbesondere über Funk. Insbesondere können hierfür RFID-Chips am Mehrkammerbeutel angeordnet werden, über welche die Temperaturmesswerte ausgelesen werden können.

Zur Überprüfung der Öffnung der Trennanordnung 330 wird nun für einen gewissen Zeitraum nur einer der beiden Heizbereiche beheizt, z. B. nur der erste Heizbereich 410. Ist die Trennanordnung 330 nicht geöffnet worden, so erwärmt die Heizfläche 410 lediglich die Flüssigkeit in der ersten Kammer 310. Wurde dagegen die Trennanordnung 330 ordnungsgemäß geöffnet, so erwärmt der Heizbereich 410 sowohl die Flüssigkeit aus der ersten Kammer 310 als auch die Flüssigkeit aus der zweiten Kammer 320, da die beiden Kammern 310 und 320 miteinander in Verbindung stehen bzw. eine gemeinsame Kammer bilden.

Hierdurch ergibt sich je nachdem, ob die Trennanordnung geöffnet wurde oder nicht, ein anderer Temperaturverlauf der Flüssigkeit. Die Überprüfung der ordnungsgemäßen Öffnung erfolgt dabei anhand eines Temperatursensors, welcher einer der beiden Kammern zugeordnet ist. Dabei kann ein Temperatursensor 415 eingesetzt werden, welcher der ersten Kammer 310 zugeordnet ist und/oder ein Temperatursensor 425, welcher der zweiten Kammer 320 zugeordnet ist. Im Ausführungsbeispiel sind dabei sowohl ein erster Sensor 415 als auch ein zweiter Sensor 425 vorgesehen. Der erste Sensor 415 ist dabei so auf der Heizfläche 400 angeordnet, dass er mit dem Bereich der ersten Kammer 310 des Mehrfachkammerbehälters 300 in Kontakt steht. Der zweite Sensor 425 ist ebenfalls so an der Heizfläche 400 angeordnet, dass er mit dem Bereich der zweiten Kammer 320 in Kontakt steht.

In einem ersten Ausführungsbeispiel der vorliegenden Erfindung kann nun überprüft werden, ob die Flüssigkeit im Bereich der zweiten Kammer 320 erwärmt wurde, obwohl nur die der ersten Kammer 310 zugeordnete erste Heizfläche 410 beheizt wurde. So kann z. B. die Temperatur im Bereich der zweiten Kammer 420 nach einer gewissen Heizzeit gemessen werden und überprüft werden, ob diese einen gewissen Grenzwert überschreitet. Ebenso ist es denkbar, zunächst einen ersten Temperaturwert und dann nach einer gewissen Heizzeit einen zweiten Temperaturwert zu bestimmen und zu überprüfen, ob die Differenz über einem gewissen Grenzwert liegt. Bleibt die Temperatur bzw. die Temperaturdifferenz im Bereich der zweiten Kammer unterhalb eines vorgegebenen Grenzwertes, so bedeutet dies, dass die Trennanordnung nicht sachgerecht geöffnet wurde.

Alternativ oder zusätzlich kann auch die Temperatur der ersten Kammer 310, welche über die erste Heizfläche 410 beheizt wird, bestimmt werden. Dabei kann z. B. abgefragt werden, ob diese Temperatur nach einer gewissen Aufheizzeit oberhalb eines Grenzwertes liegt. Ist dies der Fall, kann daraus geschlossen werden, dass die Trennanordnung 330 nicht geöffnet wurde. Ebenso ist es denkbar, anhand von zwei oder mehr Meßwerten die Aufheizgeschwindigkeit zu bestimmen. Liegt diese über einem gewissen Grenzwert, so kann darauf geschlossen werden, dass die Trennanordnung 330 nicht geöffnet wurde.

Die Überprüfungsphase, in welcher der Mehrkammerbehälter nur über einen der beiden Heizbereiche beheizt wird, kann dabei zu Beginn der Aufrüstphase der Dialysemaschine erfolgen. Vorteilhafterweise wird nach Erkennen der ordnungsgemä-βen Öffnung dann mit beiden Heizbereichen der gesamte Mehrkammerbehälter beheizt.

In einem weiteren Ausführungsbeispiel kann die ordnungsgemäße Öffnung der Trennanordnung 330 auch durch eine in die Dialysemaschine integrierte Waagezelle erfolgen, welche das Gewicht eines Flüssigkeitsbehälters, insbesondere des Mehrkammerbehälters, ermittelt, Z. B. kann es sich dabei um eine Waagezelle handeln, wie sie auch bei dem in Figur 8 gezeigten zweiten Ausführungsbeispiel eingesetzt wird. Die Waagezelle misst dabei das Gewicht des Mehrkammerbehälters. Dabei kann die Waagezelle mit der Heizfläche kombiniert werden, so dass das Gewicht des auf der Heizfläche aufliegenden Mehrkammerbeutels gemessen wird. Alternativ kann auch das Gewicht des Mehrkammerbeutels bei der in Figur 14 gezeigten Anordnung gemessen werden, indem an dem Halter zur Halterung des Mehrkammerbehälters eine Waagezelle angeordnet wird.

Die Überprüfung der ordnungsgemäßen Öffnung der Trennanordnung 330 erfolgt dabei vorteilhafterweise über die Erfassung der Gewichtsabnahme über die Zeit in dem Mehrkammerbehälter. Über die hieraus ermittelte Flussgeschwindigkeit können Rückschlüsse auf das Öffnen der Trennanordnung gezogen werden. Alternativ kann die Flussgeschwindigkeit auch durch andere Bilanzierungssysteme ermittelt werden.

In einem weiteren Ausführungsbeispiel wird ein optischer Sensor eingesetzt, welcher eine optische Eigenschaft der Flüssigkeit im Mehrfachbehälter bzw. der aus dem Mehrfachbehälter entnommenen Flüssigkeit bestimmt.

Bei dem optischen Sensor kann es sich dabei z. B. um einen Trübungssensor handeln, durch welchen eine Farbveränderung der Flüssigkeit gemessen werden kann. Vorteilhafterweise ist dabei die Einzellösung in der zweiten Kammer 320, welche keinen Abfluss 350 aufweist, mit biokompatiblen Farbstoff eingefärbt. Die Flüssigkeit in der ersten Kammer 310 mit dem Abfluss 350 ist dagegen vorteilhafterweise nicht eingefärbt. Durch das Öffnen der Trennanordnung 330 und das Durchmischen der beiden Einzellösungen ergibt sich dabei eine Farbveränderung der ersten Einzellösung 310. Dies kann nun durch eine entsprechende Trübungssensorik verifiziert werden.

Ebenso ist es denkbar, dass die Einzellösung in der Kammer 310 andere Polarisierungseigenschaften aufweist als die Gesamtlösung, welche sich durch Mischung der Einzellösungen bildet. Daher kann gegebenenfalls auch durch Bestimmung der Polarisierungseigenschaften der Flüssigkeit im Beutel bzw. der aus dem Beutel entnommenen Flüssigkeit die ordnungsgemäße Öffnung und Durchmischung der beiden Einzellösungen verifiziert werden.

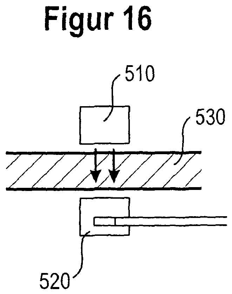

In Figur 16 ist nun ein Ausführungsbeispiel einer entsprechenden optischen Sensorik dargestellt. Dabei ist eine Lichtquelle 510 und ein optischer Sensor 520 vorgesehen. Die beiden Sensoren sind dabei so angeordnet, dass nach ordnungsgemä-βem Ankoppeln des Fluidsystems ein Fluidweg 530 zwischen der Lichtquelle 510 und dem optischen Sensor 520 angeordnet ist. Durch entsprechende Betätigung der Ventilbereiche des Fluidsystems wird nun Flüssigkeit aus dem Mehrkammerbehälter 300 in diesen Fluidweg 530 geleitet.

Durch Bestimmung einer optischen Eigenschaft der Flüssigkeit im Fluidweg 530 wird dann die ordnungsgemäße Öffnung der Trennanordnung 330 überprüft. Der Messbereich zum Messen der optischen Eigenschaft der Flüssigkeit kann dabei z. B. in einem Schlauchabschnitt der Fluidwege angeordnet werden, welcher daher beim Aufrüsten an den entsprechenden Sensorbereich der Dialysemaschine angekoppelt werden muss. Alternativ kann der Messbereich auch in die Kassette integriert werden, welche ohnehin an eine Ankoppelfläche der Dialysemaschine angekoppelt wird. Der Sensor mißt dann die optische Eigenschaft der Flüssigkeit, welche sich in einem entsprechenden Messbereich der Kassette befindet. Vorteilhafterweise sind dabei beide Seiten der Kassette in diesem Bereich transparent und weiterhin vorteilhafterweise durchsichtig.

Soll eine Farbveränderung festgestellt werden, so bestimmt der optische Sensor z.B. die Lichtstärke des durch die Flüssigkeit geleiteten Lichtes. Sollen die Polarisationseigenschaften bestimmt werden, so sendet vorteilhafterweise die Lichtquelle 510 polarisiertes Licht aus. Der optische Sensor 520 misst dann vorteilhafterweise die Veränderung der Polarisationsrichtung und/oder Polarisationsart des polarisierten Lichtes.

In einem weiteren Ausführungsbeispiel, erfolgt die Überprüfung durch eine Ultraschall-Sensorik, insbesondere über eine Reflektions-Ultraschall-Sensorik. Die sachgerechte Öffnung der Trennanordnung kann durch das Erfassen einer Reflexionscharakteristik überprüft werden.

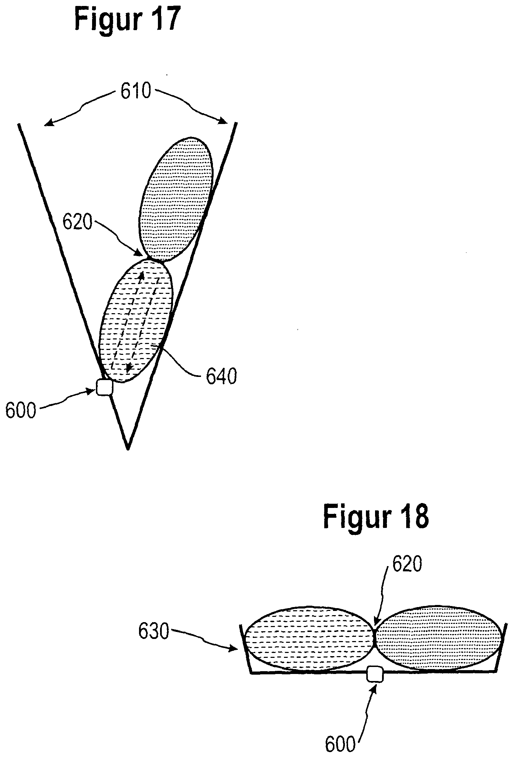

In Fig. 17 wird der Ultraschall-Sensor 600 dabei beim Ankoppeln des Mehrkammerbehälters an die Dialysemaschine an eine erste Kammer 640 des Mehrkammerbehälters angekoppelt. Durch die Bestimmung der Reflexionscharakteristik der Kammer 640 kann darauf geschlossen werden, ob die Trennanordnung 620 geöffnet wurde oder nicht, da die Größe der Kammer hiervon abhängt. Die Überprüfung kann dabei zum einen anhand eines Absolutwertes z.B. zum Füllstand in der ersten Kammer 640 erfolgen. Alternativ oder zusätzlich kann die Überprüfung auch anhand der Änderung der Reflexionscharakteristik durch Öffnen der Trennanordnung erfolgen. Das Verfahren ist daher insbesondere bei einer automatischen Öffnung der Trennanordnung durch die Dialysemaschine gut zur Überprüfung geeignet.

In Fig. 17 erfolgt die Ankopplung des Sensors 600 an die Kammer 640 dabei, indem der Sensor im Bereich einer Heizanordnung 610 angeordnet wird, an welche der Mehrkammerbeutel angekoppelt wird. Die Heizanordnung weist dabei zwei Heizplatten 610 auf, welche V-förmig angeordnet sind, wobei der Mehrkammerbeutel so aufgehängt wird, dass er zumindest auf einer der Platten möglichst großflächig aufliegt. Der Sensor 600 ist dabei auf der anderen Platte in einem unteren Bereich angeordnet, so dass die untere Kammer 640 auf dem Beutel aufliegt.

In Fig. 18 ist der Ultraschall-Sensor 600 dagegen nach dem Ankoppeln des Mehrkammerbehälters an die Dialysemaschine bei geschlossener Trennanordnung 620 nicht an den Mehrkammerbehälter angekoppelt, da er im Bereich der Trennanordnung angeordnet ist. Erst wenn diese geöffnet wird, erfolgt durch die damit verbundene Änderung in der Form des Mehrkammerbeutels eine Ankopplung des Mehrkammerbeutels an den Sensor 600. Daher liegen je nach Zustand der Trennanordnung deutlich unterschiedliche Reflexionscharakteristiken vor, so dass die Überprüfung der ordnungsgemäßen Öffnung auch anhand der Absolutwerte erfolgen kann. Eine Überprüfung anhand der Veränderung der Reflexionscharakteristik kann natürlich ebenfalls erfolgen.

In Fig. 18 ist der Sensor 600 dabei im Bereich der Heizanordnung 6130 angeordnet, auf welche der Mehrkammerbeutel aufgelegt wird. Im Ausführungsbeispiel ist der Sensor dabei in etwa mittig angeordnet, da sich in diesem Bereich die Trennanordnung befindet.

Bei den Ausführungsbeispielen in Fig. 17 und 18 kann ein Mehrkammerbeutel eingesetzt werden, wie er bereits oben beschrieben wurde.

Bei allen Ausführungsbeispielen der vorliegenden Erfindung wird die ordnungsgemäße Öffnung der Trennanordnung überprüft, bevor die Flüssigkeit aus dem Mehrkammerbeutel für die Behandlung eines Patienten eingesetzt wurde. Insbesondere bei der Peritonealdialyse erfolgt die Überprüfung daher, bevor das Dialysat aus dem Mehrkammerbeutel zum Patienten geleitet wurde. Erkennt die Dialysemaschine, dass die Trennanordnung nicht ordnungsgemäß geöffnet wurde, so gibt sie vorteilhafterweise einen entsprechenden Hinweis an den Benutzer. Der Hinweis kann dabei optisch und/oder akustisch erfolgen. Vorteilhafterweise wird dabei auf eine Anzeige ein Hinweis dargestellt, dass die Trennanordnung 330 geöffnet werden muss.

Die Maschine beginnt dabei erst dann mit der Behandlung, wenn die Trennanordnung ordnungsgemäß geöffnet wurde und damit sichergestellt ist, dass das eingesetzte Dialysat die richtige Zusammensetzung aufweist.

Die vorliegende Erfindung kann zwar gegebenenfalls auch bei der Hämodialyse eingesetzt werden. Dort sind jedoch Mehrkammerbehälter mit einer entsprechenden Trennanordnung unüblich. Zudem wird dort üblicherweise die von der Dialysemaschine vorgenommene Mischung aus mehreren Behältern durch einen Leitfähigkeitssensor überwacht.

Die vorliegende Erfindung kommt daher besonders vorteilhafterweise bei der Peritonealdialyse zum Einsatz. Bei der erfindungsgemäßen Vorrichtung handelt es sich daher vorteilhafterweise um eine Peritonealdialysemaschine. Dabei erfolgt die Überprüfung der ordnungsgemäßen Öffnung der Trennanordnung vorteilhafterweise ohne dass der Sensor in direktem Kontakt mit der Flüssigkeit in den Fluidwegen stünde.

## Patentansprüche

1. Dialysemaschine, insbesondere Peritonealdialysemaschine, an welche ein Fluidsystem mit einem Mehrkammerbehälter (300) mit mindestens zwei durch eine mechanisch zu öffnende Trennanordnung (330) getrennten Kammern (310, 320) mit Einzellösungen ankoppelbar ist, mit einer Steuerung und mindestens einem Sensor (415, 425) zur Bestimmung einer Messgröße im Fluidsystem, und mit einer Heizung (400) zum Erwärmen der Flüssigkeit im Mehrkammerbehälter, wobei die Steuerung eine Vorrichtung zur automatischen Überprüfung der sachgerechten Öffnung der Trennanordnung des Mehrkammerbehälters (300) aufweist, welche die sachgerechte Öffnung der Trennanordnung (330) eines Mehrkammerbehälters eines an die Dialysemaschine angekoppelten Fluidsystems anhand der vom Sensor (415, 425) bestimmten Messgröße automatisch überprüft,
**dadurch gekennzeichnet,**
**dass** zur Überprüfung der sachgerechten Öffnung der Trennanordnung des Mehrkammerbehälters eine erste Kammer (310) des Mehrkammerbehälters beheizt wird und eine zweite Kammer (320) nicht beheizt wird, wobei die Überprüfung durch Bestimmung einer Temperatur oder einer Temperaturveränderung im Fluidsystem erfolgt, wobei die Dialysemaschine einen der ersten Kammer (310) zugeordneten Temperatursensor (415) aufweist, über welchen die Temperatur der Flüssigkeit im Bereich der ersten Kammer bestimmbar ist, und/oder wobei die Dialysemaschine einen der zweiten Kammer (320) zugeordneten Temperatursensor (425) aufweist, über welchen die Temperatur der Flüssigkeit im Bereich der zweiten Kammer bestimmbar ist.

2. Dialysemaschine nach Anspruch 1, wobei die Vorrichtung zur automatischen Überprüfung so ausgestaltet ist, dass sie die sachgerechte Öffnung der Trennanordnung (330) des Mehrkammerbehälters (300) überprüft, bevor Flüssigkeit aus dem Mehrkammerbehälter für die Dialysebehandlung herangezogen wurde und insbesondere bevor Flüssigkeit zum Patienten geleitet wurde, wobei vorteilhafterweise die Dialysebehandlung nur dann beginnt, wenn eine sachgerechte Öffnung der Trennanordnung (330) erkannt wurde, wobei vorteilhafterweise andernfalls ein Signal an den Benutzer ausgegeben wird.

3. Dialysemaschine nach Anspruch 1 oder 2, wobei der Sensor (415, 425) so ausgestaltet ist, dass er die Messgröße im Fluidsystem ohne direkten Kontakt mit dem Fluid im Fluidsystem misst.

4. Dialysemaschine nach einem der vorangegangen Ansprüche, wobei die Vorrichtung zur automatischen Überprüfung so ausgestaltet ist, dass sie die sachgerechte Öffnung der Trennanordnung (330) des Mehrkammerbehälters anhand der Veränderung der vom Sensor (415, 425) bestimmten Messgröße über die Zeit automatisch überprüft.

5. Dialysemaschine nach einem der vorangegangen Ansprüche, wobei die Vorrichtung zur automatischen Überprüfung der sachgerechten Öffnung der Trennanordnung (330) des Mehrkammerbehälters so ausgestaltet ist, dass sie auch die sachgerechte Mischung der Einzellösungen überprüft.

6. Dialysemaschine nach einem der vorangegangen Ansprüche gekennzeichnet, dass die Dialysemaschine Mittel zur selbsttätigen Öffnung der Trennanordnung aufweist.

7. Dialysemaschine nach Anspruch 6, wobei Mittel zur selbsttätigen Öffnung der Trennanordnung Mittel zum Druckaufbau in mindestens einer Kammer des Mehrkammerbehälters aufweisen, wobei der Druckaufbau insbesondere über das Einleiten von Pressluft oder das Pumpen von Flüssigkeit in die Kammer erfolgt.

8. Dialysemaschine nach Anspruch 6 oder 7, wobei die Steuerung eine Vorrichtung zur automatischen Überprüfung der sachgerechten Öffnung der Trennanordnung des Mehrkammerbehälters (300) aufweist, welche so ausgestaltet ist, dass sie die sachgerechte Öffnung der Trennanordnung (330) durch die Mittel zur selbsttätigen Öffnung überprüft, insbesondere anhand eines Druckes und/oder eines Druckverlaufs und/oder einer Füllmenge.

9. Verfahren zum Aufrüsten einer Dialysemaschine, an welche ein Fluidsystem mit einem Mehrkammerbehälter mit mindestens zwei durch eine mechanisch zu öffnende Trennanordnung getrennten Kammern mit Einzellösungen ankoppelbar ist, mit einer Steuerung und mindestens einem Sensor zur Bestimmung einer Messgröße im Fluidsystem, und mit einer Heizung (400) zum Erwärmen der Flüssigkeit im Mehrkammerbehälter, wobei die Steuerung eine Vorrichtung zur automatischen Überprüfung der sachgerechten Öffnung der Trennanordnung des Mehrkammerbehälters aufweist, welche die sachgerechte Öffnung der Trennanordnung eines Mehrkammerbehälters eines an die Dialysemaschine angekoppelten Fluidsystems anhand der vom Sensor bestimmten Messgröße automatisch überprüft, mit den Schritten:
Ankoppeln eines Fluidsystems mit einem Mehrkammerbehälter (300) mit mindestens zwei durch eine mechanisch zu öffnende Trennanordnung (330) getrennten Kammern (310, 320),
Bestimmen einer Messgröße im Fluidsystem über einen Sensor (415, 425) der Dialysemaschine,
Überprüfen der sachgerechten Öffnung der Trennanordnung (330) des Mehrkammerbehälters (300) eines an die Dialysemaschine angekoppelten Fluidsystems anhand der Messgröße,
**dadurch gekennzeichnet,**
**dass** zur Überprüfung der sachgerechten Öffnung der Trennanordnung des Mehrkammerbehälters eine erste Kammer (310) des Mehrkammerbehälters beheizt wird und eine zweite Kammer (320) nicht beheizt wird, wobei die Überprüfung durch Bestimmung einer Temperatur oder einer Temperaturveränderung im Fluidsystem erfolgt.

10. Verfahren nach Anspruch 9, mit dem Schritt:
Auslösen eines Alarms und/oder Verhinderung eines Starts der Behandlung, wenn die Messgröße nicht einer bei einer sachgerechten Öffnung der Trennanordnung (330) erwarteten Messgröße entspricht, insbesondere wenn die Messgröße außerhalb eines vorgegebene Bereichs oder unter- oder oberhalb eines Grenzwertes liegt oder der Verlauf der Messgröße nicht einem erwarteten Verlauf entspricht.

11. Verfahren nach einem der Ansprüche 9 oder 10 mit dem Schritt:
selbsttätiges Öffnen der Trennanordnung durch die Dialysemaschine.

12. Verfahren nach einem der Ansprüche 9 bis 11 zum Aufrüsten einer Dialysemaschine nach einem der Ansprüche 1 bis 8.

## Claims

1. Dialysis machine, in particular a peritoneal dialysis machine, to which a fluid system having a multi-chamber container (300) with at least two chambers (310, 320) with individual solutions separated by a partition arrangement (330) to be opened mechanically can be coupled having a controller and at least one sensor (415, 425) for the determination of a measured variable in the fluid system, and comprising a heating (400) for the heating of the fluid in the multi-chamber container; wherein the controller has an apparatus for the automatic checking of the proper opening of the partition arrangement of the multi-chamber container (300) which automatically checks the proper opening of the partition arrangement (330) of a multi-chamber container of a fluid system coupled to the dialysis machine with reference to the measured variable determined by the sensor (415, 425)
**characterized in that**
a first chamber (310) of the multi-chamber container is heated for the checking of the proper opening of the partition arrangement of the multi-chamber container and a second chamber (320) is not heated; wherein the checking takes place by determination of a temperature or of a temperature change in the fluid system, wherein the dialysis machine has a temperature sensor (415) which is associated with the first chamber (310) and via which the temperature of the fluid can be determined in the region of the first chamber; and/or wherein the dialysis machine has a temperature sensor (425) which is associated with the second chamber (320) and via which the temperature of the fluid in the region of the second chamber can be determined.

2. Dialysis machine in accordance with claim 1, wherein the apparatus for the automatic checking checks the proper opening of the partition arrangement (330) of the multi-chamber container (300) before fluid is removed from the multi-chamber container for the dialysis treatment and in particular before fluid was conducted to the patient; wherein the dialysis treatment advantageously only begins when a proper opening of the partition arrangement(330) was recognized; wherein advantageously otherwise a signal is output to the user.

3. Dialysis machine in accordance with either of claims 1 or 2, wherein the sensor (415, 425) measures the measured variable in the fluid system without direct contact with the fluid in the fluid system.

4. Dialysis machine in accordance with one of the preceding claims, wherein the apparatus for the automatic checking automatically checks the proper openings of the partition arrangement (330) of the multi-chamber container with reference to the change in the measured variable determined by the sensor (415, 425) over time.

5. Dialysis machine in accordance with one of the preceding claims, wherein the apparatus for the automatic checking of the proper opening of the partition arrangement (330) of the multi-chamber container also checks the proper mixing of the individual solutions.

6. Dialysis machine in accordance with one of the preceding claims, wherein the dialysis machine has means for the automatic opening of the partition arrangement.

7. Dialysis machine in accordance with claim 6, wherein means for the automatic opening of the partition arrangement have means for the pressure build-up in at least one chamber of the multi-chamber container; wherein the pressure build-up in particular takes place via the introduction of compressed air or the pumping of fluid into the chamber.

8. Dialysis machine in accordance with either of claims 6 or 7, wherein the controller has an apparatus for the automatic checking of the proper opening of the partition arrangement of the multi-chamber container (300) which checks the proper opening of the partition arrangement (330) by the means for the automatic opening, in particular with reference to a pressure and/or to a pressure development and/or a filling quantity.

9. Method for the activation of a dialysis machine, to which a fluid system having a multi-chamber container to at least two chambers separated by a partition arrangement to be opened mechanically can be coupled, having a controller and at least one sensor for the determination of a measured variable in the fluid system, and comprising a heating (400) for the heating of the fluid in the multi-chamber container, wherein the controller has an apparatus for the automatic checking of the proper opening of the partition arrangement of the multi-chamber container, which automatically checks the proper opening of a multi-chamber container of a fluid system coupled to the dialysis machine with reference to the measured variable determined by the sensor, comprising the steps:
coupling a fluid system having a multi-chamber container (300) to at least two chambers (310, 320) separated by a partition arrangement (330) to be opened mechanically,
determining a measured variable in the fluid system via a sensor (415, 425) of the dialysis machine,
checking the proper opening of the partition arrangement (330) of the multi-chamber container (300) of a fluid system coupled to the dialysis machine with reference to the measured variable,
**characterized in that**
a first chamber (310) of the multi-chamber container is heated for the checking of the proper opening of the partition arrangement of the multi-chamber container and a second chamber (320) is not heated; wherein the checking takes place by determination of a temperature or of a temperature change in the fluid system.

10. Method in accordance with claim 9, comprising the step:
triggering an alarm and/or prevention of a start of the treatment if the measured variable does not correspond to a measured value expected on a proper opening of the partition arrangement (330), in particular when the measured variable is outside a preset range or below or above a limit value or if the development of the measured variable does not correspond to an expected development.

11. Method in accordance with one of the claims 9 or 10, comprising the step:
automatic opening of the partition arrangement by the dialysis machine.

12. Method in accordance with one of the claims 9 to 11 for for the activation of a dialysis machine in accordance with one of claims 1 to 8.

## Revendications

1. Machine de dialyse, en particulier machine de dialyse péritonéale, à laquelle peut être couplé un système de fluide doté d'un contenant à plusieurs compartiments (300) avec au moins deux compartiments (310, 320) comportant des solutions individuelles et séparés par un ensemble de séparation (330) à ouverture mécanique, doté d'une commande et d'au moins un capteur (415, 425) destiné à déterminer une grandeur de mesure dans le système de fluide, et doté d'un chauffage (400) destiné à chauffer le liquide situé dans le contenant à plusieurs compartiments, la commande présentant un dispositif de vérification automatique de l'ouverture appropriée de l'ensemble de séparation du contenant à plusieurs compartiments (300), lequel dispositif vérifie automatiquement l'ouverture appropriée de l'ensemble de séparation (330) d'un contenant à plusieurs compartiments d'un système de fluide couplé à la machine de dialyse à l'aide de la grandeur de mesure déterminée par le capteur (415, 425),
**caractérisée en ce que**
pour la vérification de l'ouverture appropriée de l'ensemble de séparation du contenant à plusieurs compartiments, un premier compartiment (310) du contenant à plusieurs compartiments est chauffé et un second compartiment (320) n'est pas chauffé, la vérification s'effectuant par la détermination d'une température ou d'une modification de température dans le système de fluide, la machine de dialyse présentant un capteur de température (415) associé au premier compartiment (310), par le biais duquel la température du liquide dans la zone du premier compartiment peut être déterminée, et/ou la machine de dialyse présentant un capteur de température (425) associé au second compartiment (320), par le biais duquel la température du liquide dans la zone du second compartiment peut être déterminée.

2. Machine de dialyse selon la revendication 1, dans laquelle le dispositif de vérification automatique est configuré de manière à vérifier l'ouverture appropriée de l'ensemble de séparation (330) du contenant à plusieurs compartiments (300) avant que du liquide provenant du contenant à plusieurs compartiments soit mis à contribution pour le traitement de dialyse et en particulier avant que du liquide soit acheminé au patient, dans laquelle, de manière avantageuse, le traitement de dialyse ne commence que quand une ouverture appropriée de l'ensemble de séparation (330) a été détectée, dans laquelle, de manière avantageuse, un signal destiné à l'utilisateur est sinon émis.

3. Machine de dialyse selon la revendication 1 ou 2, dans laquelle le capteur (415, 425) est configuré de manière à mesurer la grandeur de mesure dans le système de fluide sans contact direct avec le fluide situé dans le système de fluide.

4. Machine de dialyse selon l'une des revendications précédentes, dans laquelle le dispositif de vérification automatique est configuré de manière à vérifier automatiquement l'ouverture appropriée de l'ensemble de séparation (330) du contenant à plusieurs compartiments à l'aide de la modification dans le temps de la grandeur de mesure déterminée par le capteur (415, 425).

5. Machine de dialyse selon l'une des revendications précédentes, dans laquelle le dispositif de vérification automatique de l'ouverture appropriée de l'ensemble de séparation (330) du contenant à plusieurs compartiments est configuré de manière à également vérifier le mélange approprié des solutions individuelles.

6. Machine de dialyse selon l'une des revendications précédentes, dans laquelle la machine de dialyse présente des moyens d'ouverture automatique de l'ensemble de séparation.

7. Machine de dialyse selon la revendication 6, dans laquelle des moyens d'ouverture automatique de l'ensemble de séparation présentent des moyens de montée en pression dans au moins un compartiment du contenant à plusieurs compartiments, la montée en pression s'effectuant en particulier par l'introduction d'air comprimé ou le pompage de liquide dans le compartiment.

8. Machine de dialyse selon la revendication 6 ou 7, dans laquelle la commande présente un dispositif de vérification automatique de l'ouverture appropriée de l'ensemble de séparation du contenant à plusieurs compartiments (300), lequel dispositif est configuré de manière à vérifier l'ouverture appropriée de l'ensemble de séparation (330) par les moyens d'ouverture automatique, en particulier à l'aide d'une pression et/ou d'une variation de pression et/ou d'une quantité de remplissage.

9. Procédé de préparation d'une machine de dialyse à laquelle peut être couplé un système de fluide doté d'un contenant à plusieurs compartiments avec au moins deux compartiments comportant des solutions individuelles et séparés par un ensemble de séparation à ouverture mécanique, doté d'une commande et d'au moins un capteur destiné à déterminer une grandeur de mesure dans le système de fluide, et doté d'un chauffage (400) destiné à chauffer le liquide situé dans le contenant à plusieurs compartiments, la commande présentant un dispositif de vérification automatique de l'ouverture appropriée de l'ensemble de séparation du contenant à plusieurs compartiments, lequel dispositif vérifie automatiquement l'ouverture appropriée de l'ensemble de séparation d'un contenant à plusieurs compartiments d'un système de fluide couplé à la machine de dialyse à l'aide de la grandeur de mesure déterminée par le capteur, comportant les étapes :
couplage d'un système de fluide à un contenant à plusieurs compartiments (300) doté d'au moins deux compartiments (310, 320) séparés par un ensemble de séparation (330) à ouverture mécanique,
détermination d'une grandeur de mesure dans le système de fluide par le biais d'un capteur (415, 425) de la machine de dialyse,
vérification de l'ouverture appropriée de l'ensemble de séparation (330) du contenant à plusieurs compartiments (300) d'un système de fluide couplé à la machine de dialyse à l'aide de la grandeur de mesure,
**caractérisé en ce que**
pour la vérification de l'ouverture appropriée de l'ensemble de séparation du contenant à plusieurs compartiments, un premier compartiment (310) du contenant à plusieurs compartiments est chauffé et un second compartiment (320) n'est pas chauffé, la vérification s'effectuant par la détermination d'une température ou d'une modification de température dans le système de fluide.

10. Procédé selon la revendication 9, comportant l'étape :
déclenchement d'une alarme et/ou empêchement d'un démarrage du traitement quand la grandeur de mesure ne correspond pas à une grandeur de mesure attendue lors d'une ouverture appropriée de l'ensemble de séparation (330), en particulier quand la grandeur de mesure est en dehors d'une plage prédéfinie ou en dessous ou au-dessus d'une valeur limite ou que la variation de la grandeur de mesure ne correspond pas à une variation attendue.

11. Procédé selon l'une des revendications 9 ou 10, comportant l'étape :
ouverture automatique de l'ensemble de séparation par la machine de dialyse.

12. Procédé selon l'une des revendications 9 à 11 pour la préparation d'une machine de dialyse selon l'une des revendications 1 à 8.
